# EUROPEAN PATENT SPECIFICATION

(11) **EP 2 817 632 B1**
(45) Date of publication and mention of the grant of the patent: **25.04.2018**
(21) Application number: 13705485.4
(22) Date of filing: 22.02.2013
(51) Int. Cl.: G01N 33/68

(54) **NEW DUAL BIOMARKER OF NEURODEGENERATION AND OF NEUROREGENERATION**
NEUE DUALE BIOMARKER ZUR NEURODEGENERATION UND NEUROREGENERATION
NOUVEAUX BIOMARQUEURS DOUBLES DE LA NEURODÉGÉNÉRESCENCE ET DE LA NEURORÉGÉNÉRATION

(30) Priority: 22.02.2012 EP 12305207; 17.09.2012 EP 12306122
(43) Date of publication of application: 31.12.2014
(73) Proprietor: CENTRE NATIONAL DE LA RECHERCHE SCIENTIFIQUE, 75794 Paris Cedex 16 (FR); College de France, 75231 Paris Cedex 05 (FR); Ecole Normale Supérieure, 75230 Paris Cedex 05 (FR); INSERM - Institut National de la Santé et de la Recherche Médicale, 75654 Paris Cedex (FR); Université Pierre et Marie Curie (Paris 6), 75005 Paris (FR)
(72) Inventor: MARIN, Philippe, F-34980 Saint Gely du Fesc (FR); THOUVENOT, Eric, F-34980 Saint Gely du Fesc (FR); NGUYEN, Geneviève, F-75015 Paris (FR); GROSZER, Matthias, F-75005 Paris (FR)
(74) Representative: LLR
(86) International application number: PCT/EP2013/053538
(87) International publication number: WO 2013/124406

(56) References cited:
- COUSIN, C.; BRACQUART, D.; CONTREPAS, A.; CORVOL, P.; MULLER, L.; NGUYEN, G.: "Soluble form of the (pro)renin receptor generated by intracellular cleavage by furin is secreted in plasma", HYPERTENSION, vol. 53, 2009, pages 1077-1082, XP002668816, cited in the application
- A. CONTREPAS ET AL: "A role of the (pro)renin receptor in neuronal cell differentiation", AMERICAN JOURNAL OF PHYSIOLOGY. REGULATORY, INTEGRATIVE AND COMPARATIVE PHYSIOLOGY, vol. 297, no. 2, 27 May 2009 (2009-05-27), pages R250-R257, XP055026783, ISSN: 0363-6119, DOI: 10.1152/ajpregu.90832.2008
- Do-Koyanagi: "Human soluble (Pro)renin Receptor Assay Kit - IBL", , 10 November 2011 (2011-11-10), XP055056686, Retrieved from the Internet: URL:http://www.ibl-america.com/pdf/newIBLe lisa/27782.pdf [retrieved on 2013-03-15]
- F. SUN ET AL: "Neuroproteomics Approaches to Decipher Neuronal Regeneration and Degeneration", MOLECULAR & CELLULAR PROTEOMICS, vol. 9, no. 5, 17 December 2009 (2009-12-17), pages 963-975, XP055026934, ISSN: 1535-9476, DOI: 10.1074/mcp.R900003-MCP200
- EMMANUELLE MOUTON-BARBOSA ET AL: "In-depth Exploration of Cerebrospinal Fluid by Combining Peptide Ligand Library Treatment and Label-free Protein Quantification", MOLECULAR & CELLULAR PROTEOMICS, vol. 9, no. 5, 1 May 2010 (2010-05-01), pages 1006-1021, XP055026634,
- ERIC THOUVENOT ET AL: "Quantitative proteomic analysis reveals protein expression changes in the murine neuronal secretome during apoptosis", JOURNAL OF PROTEOMICS, vol. 77, 1 December 2012 (2012-12-01), pages 394-405, XP055049183, ISSN: 1874-3919, DOI: 10.1016/j.jprot.2012.09.013

## Description

The present invention relates to a new dual biomarker of neurodegeneration and of neuroregeneration.

The cerebrospinal fluid (CSF) is a sample of choice for biomarker discovery by proteomic approaches. The human CSF proteome has been extensively characterized (Zougman, A., Pilch, B., Podtelejnikov, A., Kiehntopf, M., Schnabel, C., Kumar, C., and Mann, M. (2008) Integrated Analysis of the Cerebrospinal Fluid Peptidome and Proteome. J Proteome Res 7, 386-399 ; Mouton-Barbosa, E., Roux-Dalvai, F., Bouyssie, D., Berger, F., Schmidt, E., Righetti, P. G., Guerrier, L., Boschetti, E., Burlet-Schiltz, O., Monsarrat, B., and dePeredo, A. G. (2010) In-depth exploration of cerebrospinal fluid by combining peptide ligand library treatment and label-free protein quantification. Mol Cell Proteomics 9, 1006-1021 ; Schutzer, S. E., Liu, T., Natelson, B. H., Angel, T. E., Schepmoes, A. A., Purvine, S. O., Hixson, K. K., Lipton, M. S., Camp, D. G., Coyle, P. K., Smith, R. D., and Bergquist, J. (2010) Establishing the proteome of normal human cerebrospinal fluid. PLoS One 5, e10980 ; Thouvenot, E., Urbach, S., Dantec, C., Poncet, J., Seveno, M., Demettre, E., Jouin, P., Touchon, J., Bockaert, J., and Marin, P. (2008) Enhanced detection of CNS cell secretome in plasma protein-depleted cerebrospinal fluid. J Proteome Res 7, 4409-4421 ; Pan, S., Zhu, D., Quinn, J. F., Peskind, E. R., Montine, T. J., Lin, B., Goodlett, D. R., Taylor, G., Eng, J., and Zhang, J. (2007) A combined data set of human cerebrospinal fluid proteins identified by multi-dimensional chromatography and tandem mass spectrometry. Proteomics 7, 469-473.) and is still actively investigated to identify relevant biomarkers and therapeutic targets for neurodegenerative diseases (Kroksveen, A. C., Opsahl, J. A., Aye, T. T., Ulvik, R. J., and Berven, F. S. (2011) Proteomics of human cerebrospinal fluid: discovery and verification of biomarker candidates in neurodegenerative diseases using quantitative proteomics. J Proteomics 74, 371-388). However, these efforts have been met with limited success (Hsich, G., Kenney, K., Gibbs, C. J., Lee, K. H., and Harrington, M. G. (1996) The 14-3-3 brain protein in cerebrospinal fluid as a marker for transmissible spongiform encephalopathies. N Engl J Med 335, 924-930) due to the difficulty in identifying in CSF samples proteins that originate from neurons and that actually reflect neuronal dysfunction or death. Although apoptosis is a major mechanism of neuronal death in many neurodegenerative disorders (Ekshyyan, O., and Aw, T. Y. (2004) Apoptosis: a key in neurodegenerative disorders. Curr Neurovasc Res 1, 355-371), apoptotic neurons are often sparse in the brain of patients, making even more uncertain the identification of biomarkers of neuronal apoptosis in CSF. Sun and Cavalli (2009) reviews neuroproteomics approaches to decipher neuronal regeneration and degeneration, and discloses inter alia that CRMP-2 is decreased upon nerve injury and at the same time is important for axon outgrowth and regeneration. Another approach consists in differential proteomic analysis of proteins released in the extracellular medium by "healthy" neurons and by neurons degenerating by apoptosis. Cerebellar granule neurons (CGNs) in primary culture are one of the best characterized *in vitro* models of the programmed cell death that occurs during normal CNS development and shares many features with the neuronal apoptosis observed in neurodegenerative disorders. These neurons degenerate by apoptosis without detectable induction of necrosis when exposed to a physiological K+ concentration (5 mM) in the absence of serum (Gallo, V., Kingsbury, A., Balazs, R., and Jorgensen, O. S. (1987) The role of depolarization in the survival and differentiation of cerebellar granule cells in culture. J Neurosci 7, 2203-2213). Apoptosis of CGNs induced by serum deprivation can be prevented by exposure of the cultures to a depolarizing concentration of K+ (30 mM) or to neurotrophins, such as insulin-like growth factor-1 (IGF-1) (Gallo, V., Kingsbury, A., Balazs, R., and Jorgensen, O. S. (1987); D'Mello, S. R., Galli, C., Ciotti, T., and Calissano, P. (1993) Induction of apoptosis in cerebellar granule neurons by low potassium: inhibition of death by insulin-like growth factor I and cAMP. Proc Natl Acad Sci U S A 90, 10989-10993). A transcriptomic analysis has revealed that one third of the transcripts that are differentially regulated during the early phase of CGN apoptosis encode extracellular proteins (Desagher, S., Severac, D., Lipkin, A., Bernis, C., Ritchie, W., Le Digarcher, A., and Journot, L. (2005) Genes regulated in neurons undergoing transcription dependent apoptosis belong to signaling pathways rather than the apoptotic machinery. J Biol Chem 280, 5693-5702), thus underscoring the relevance of analyzing the CGN secretome for characterizing diffusible biomarkers of neuronal apoptosis.

Stable isotope labeling by/with amino acids in cell culture (SILAC) is a technique based on mass spectrometry that detects differences in protein abundance among samples using nonradioactive isotopic labeling.

It allows detecting quantitative variations in protein in cells, but its accuracy in neuronal cultures, based on the comparison of labeled and unlabeled samples, is limited by the incomplete protein labeling in neurons, which do not divide in culture such as cerebellar granule neurons (CGN).

Neuroregeneration to the opposite to neurodegeneration refers to the neuronal multiplication or differentiation or the synaptogenesis. Such mechanisms may include generation of new neurons, glia, axons, myelin, or synapses. So far, only a few biomarkers of neurogenesis are known. PRR is a protein corresponding to renin and prorenin receptor. It has been cloned by G. Nguyen Nguyen G, Delarue F, Burcklé C, Bouzhir L, Giller T, Sraer J-D Pivotal role of the renin/prorenin receptor in angiotensin II production and cellular responses to renin. J Clin Invert 109: 1417-1427, 2002) and is an essential component of the renin-angiotensin system. PRR is highly expressed in the SNC during human development and in the adult, mainly in neurons. It is a receptor having only one transmembrane receptor, the extracellular domain of which can be liberated in the extracellular medium after cleavage by furine in the trans-Golgi compartment (soluble form of PRR: sPRR). The renin and pro renin binding to PRR increases their catalytic activity and activates intracellular signalling pathways including MAP-kinases ERK1/2 and phosphatidyl inositol 3-kinase (PI3-K).

sPRR can be detected in plasma using standard Elisa. sPRR has also been detected in CSF by tandem-mass spectrometry in one of the most comprehensive proteomic analyses of human CSF available Mouton-Barbosa, E., Roux-Dalvai, F., Bouyssie, D., Berger, F., Schmidt, E., Righetti, P. G., Guerrier, L., Boschetti, E., Burlet-Schiltz, O., Monsarrat, B., and dePeredo, A. G. (2010) In-depth exploration of cerebrospinal fluid by combining peptide ligand library treatment and label-free protein quantification. Mol Cell Proteomics 9, 1006-1021.

The increasing frequency of neurodegenerative diseases and the lack of early and specific diagnostic and prognostic biomarkers necessitate the discovery of such new biomarkers of these diseases as well as biomarker for neurogenesis.

The aim of the invention is to provide a diagnostic and prognostic biomarker of neurodegenerative diseases.

Another aim of the invention is to provide a diagnostic and prognostic biomarker for the neuroregeneration.

Another aim of the invention is to provide a method liable to quantify the level of said biomarker in neurons, which do not divide in culture.

The present invention relates to the i*n vitro* use of the secreted, extra cellular form of the soluble part of (pro)renin receptor as set forth by SEQ ID NO:1 or fragments thereof, the level of which has been determined in a sample of a biological fluid previously collected in a patient or an animal, as an early dual biomarker of neurodegeneration and neuroregeneration.

The (pro)renin receptor (full length) is set forth by SEQ ID NO:3 and is constituted of a peptide signal, an extra cellular domain as set forth by SEQ ID NO:2, containing the soluble part of the (pro)renin receptor of SEQ ID NO:1, a transmembrane domain and a cytoplasmic domain.

After cleavage at the furin cleavage site R275A/KT/R278A of the extra cellular domain, the soluble part of (pro)renin receptor is thus secreted under an extra cellular form, set forth by SEQ ID NO:1, without the signal peptide.

The term "fragment" in all the description means a part of sPRR (SEQ ID NO:1) having a length of at least 6 amino acids, preferably at least 10 amino acids, more preferably at least 20 amino acids, even more preferably at least 100 amino acids provided that said fragment has kept the biological activity of sPRR (SEQ ID NO:1).

The inventors have unexpectedly found that sPRR protein or fragments thereof, i.e. the secreted, extra cellular form of the soluble part of (pro)renin receptor as set forth by SEQ ID NO:1 or fragments thereof, was differentially expressed in supernatants of apoptotic and surviving neurons, as determined by a SILAC method based on two different isotope labelings (L-[¹³C6]arginine and L-[²H4]lysine, or L-[¹³C6-¹⁵N4]arginine and L-[¹³C6-¹⁵N2]lysine), in combination with liquid chromatography coupled to Fourier transform tandem mass spectrometry (LC-FT-MS/MS).

This method allows identifying quantitative variations in protein secretion in particular by primary cultures of cerebellar granule neurons (CGNs) during apoptosis, without encountering the problem of accuracy of the SILAC method arising from the incomplete protein labeling in neurons, which do not divide in culture.

Thus said quantitative variations in protein secretion that have been identified allows using said proteins as an early dual biomarker of neurodegeneration and neuroregeneration in a sample of a biological fluid by identification and quantification of said biomarker in said biological fluid.

The term "neurodegeneration" means neuronal damages such as synapse loss, or neuronal death. It also covers psychiatric pathologies.

The term "neuroregeneration" means neuronal protection, neuronal multiplication, neuronal differentiation or synaptogenesis depending on the level of said protein in said biological fluid.

The biological fluid can be the extracellular medium of any cell culture, in particular neurons in primary culture but also the blood, the plasma, the serum, urine or the cerebrospinal fluid (CSF).

The term "sample" in all the description refers to a part of said biological fluid.

The term "patient" in all the description refers to a human.

The expression "in a sample of a biological fluid previously collected in a patient or an animal" in all the description, means that the collecting step of said biological fluid is excluded from the method or process of the invention.

The expression "dual biomarker" means that said protein as set forth by SEQ ID NO:1 or fragments thereof, is not only involved in neurodegeneration but also in neuroregeneration.

Thus another advantage of the invention is to provide a same biomarker liable to be used as neurodegeneration biomarker and neuroregeneration biomarker.

The word "early" means that said biomarker can be identified at a very early stage of a neurodegenerative disease, i.e. before onset of clinical symptoms which are observed when massive neuronal death has occurred and the efficacy of therapeutic intervention is compromised.

In other words, "early" means "pre-symptomatic". This biomarker of neurodegeneration allows also to make a prognostic of MCI and further to evaluate the evolution of MCI toward Alzheimer's disease.

In an advantageous embodiment, the present invention relates to the use of the secreted, extra cellular form of the soluble part of (pro)renin receptor as set forth by SEQ ID NO:1 or fragments thereof, the level of which as been determined in a biological fluid of a patient or an animal, as an early dual biomarker of neurodegeneration and neuroregeneration.

Thus, in this embodiment, the use of SEQ ID NO: 1 is carried out *ex vivo.*

In an advantageous embodiment, the present invention relates to the *in vitro* use of the secreted, extra cellular form of the soluble part of (pro)renin receptor as set forth by SEQ ID NO:1 or fragments thereof, the level of which has been determined in a sample of a biological fluid previously collected in a patient or an animal, as an early dual biomarker of neurodegeneration and neuroregeneration, wherein said biological fluid is the cerebrospinal fluid or the extracellular medium of any neuronal cell in primary culture, in particular in cerebellar granule neurons in primary culture.

The inventors have unexpectedly found that the protein as set forth by SEQ ID NO:1 or fragments thereof, present in particular in the extracellular medium of a neuronal cell in primary culture such as a granule neurons from cerebellum and in particular present in the cerebrospinal fluid has a level of expression that is modulated in function of pathologies such as neurodegenerative diseases or in case of neuroregeneration.

The determination or quantification of the level of SEQ ID NO:1 and comparison with a control sample allows identifying an early stage of neurodegeneration or neuroregeneration is said neuronal cell in primary culture, in particular in said cerebellar granule neurons, and in particular in said cerebrospinal fluid. To characterize the quantitative variations in sPRR secretion by primary cultures of CGNs during apoptosis, the stable isotope labeling by amino acids in cell culture (SILAC) technology in combination with liquid chromatography coupled to Fourier transform tandem mass spectrometry (LC-FT-MS/MS) has been used.

In an advantageous embodiment, the present invention relates to the use of the secreted, extra cellular form of the soluble part of (pro)renin receptor as set forth by SEQ ID NO:1 or fragments thereof, the level of which has been determined in a biological fluid, as an early dual biomarker of neurodegeneration and neuroregeneration, wherein said biological fluid is the cerebrospinal fluid or the extracellular medium of any neuronal cell, in particular in cerebellar granule neurons.

Thus, in this embodiment, the use of SEQ ID NO:1 is carried out *ex vivo.*

In another aspect, the present invention relates to a process of identification of an early dual biomarker of neurodegeneration and neuroregeneration, by determination, by the SILAC method, in mouse cerebellar granule neurons, of the ratio:
a) between the level of a protein present in mouse cerebellar granule neurons deprived of serum in presence of a depolarizing concentration of potassium or of neurotrophin such as insulin-like growth factor-1 and between the level of said protein in mouse cerebellar granule neurons deprived of serum in presence of a physiological concentration of potassium,
and of the ratio:
b) between the level of said protein in mouse cerebellar granule neurons in a condition to evaluate and between the level of a protein present in mouse cerebellar granule neurons in normal or healthy condition,
and said protein being a dual biomarker if said ratio measured in steps a) and b) is above thresholds value providing a *p* value of 0.01 for normalized peptide ratio, based on significance B, as calculated using the Maxquant software.

When mouse cerebellar granule neurons are deprived of serum in presence of a physiological concentration of potassium, such as 5 mM, they degenerate by apoptosis without detectable induction of cell necrosis.

Apoptosis of CGNs induced by serum deprivation can be prevented by exposure of the cultures to a depolarizing concentration of potassium (30 mM) or to neurotrophins, such as insulin-like growth factor-1 (IGF-1) giving neurons in conditions of normal or healthy surviving.

Thus when said ratio measured in steps a) and b) is above thresholds value providing a *p* value of 0.01 for normalized peptide ratio, based on significance B, as calculated using the Maxquant sofware, that means that the level of the protein has been decreased in said neurons deprived of serum in presence of a physiological concentration of potassium compared with the one with a depolarizing concentration of potassium or IGF-1 and thus is representative of neurodegeneration or a neurodegenerative disease giving thus a neurodegeneration biomarker.

Said ratio has been obtained 12 hours after apoptosis induction and is therefore representative of an early stage of the neurodegeneration process, and thus is an early biomarker of neurodegeneration.

That means that a ratio higher than the threshold ratio corresponding to a p value of 0.01 might reflect a more advanced neurodegenerative process.

When the ratio defined in step b) is higher than 1, that means that the level of the protein measured in a condition to test is higher than the one of said protein measured in normal or healthy conditions and is thus representative of neuronal proliferation or neuroregeneration.

Said ratio is therefore representative of an early stage of the neuronal proliferation or neuroregeneration process, and thus is an early biomarker of neuronal proliferation or neuroregeneration.

In an advantageous embodiment, the present invention relates to a process of identification of an early dual biomarker of neurodegeneration and neuroregeneration, wherein said protein is the secreted, extra cellular form of the soluble part of the (pro)renin receptor as set forth by SEQ ID NO:1 or fragments thereof, and CGN cultures have been exposed to a depolarizing concentration of potassium, and wherein said ratio measured in step a) and in step b) is higher that 1, in particular 4.79.

In an advantageous embodiment, the present invention relates to a process of identification of an early dual biomarker of neurodegeneration and neuroregeneration, wherein said protein is the secreted, extra cellular form of the soluble part of the (pro)renin receptor as set forth by SEQ ID NO:1 or fragments thereof, and CGN cultures have been exposed to IGF-1 and said ratio in step a) and in step b) is higher that 1, in particular 2.79. Disclosed are the secreted, extra cellular form of the soluble part of the (pro)renin receptor as set forth by SEQ ID NO:1 or fragments thereof, the level of which has been determined *in vitro* in a sample of a biological fluid previously collected in a patient or an animal,
for its use in the diagnosis and/or in the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis,
for its use in the follow up of a treatment against a neurodegenerative disease in a patient afflicted by a neurodegenerative disease, in particular Alzheimer's disease, dementia with Lewy bodies, fronto-temporal dementia, Parkinson's disease, amyotrophic lateral sclerosis, and/or
for the its use in follow up of a treatment stimulating the neuroregeneration, and/or
for its use in the screening of new drugs liable to treat neurodegenerative pathologies, in particular Alzheimer's disease, dementia with Lewy bodies, fronto-temporal dementia, Parkinson's disease, amyotrophic lateral sclerosis, and/or
for its use in the diagnosis and/or the prognosis of tumoral and/or proliferative disorders of the central nervous system, in particular glioblastomas, and /or
for its use in the follow-up of tumors of the central nervous system after surgery and/or chemotherapy,
said level being modulated with respect to a sample of said biological fluid previously collected in a control patient or a control animal.

The inventors have unexpectedly found that the identification and quantification of said secreted, extra cellular form of the soluble part of the (pro)renin receptor as set forth by SEQ ID NO: 1 or fragments thereof, in said biological fluid provides:
a biomarker of neurodegeneration and is indicative of neurodegenerative disease or of tumoral and/or proliferative disorders
as well as a biomarker of neuroregeneration,

The expression "for its use in the diagnosis ... of a neurodegenerative disease or of a neurodegenerative disease stage in a patient liable to be afflicted by a neurodegenerative disease" means that the determination of the level of the secreted, extra cellular form of the soluble part of the (pro)renin receptor as set forth by SEQ ID NO:1 or fragments thereof, either alone or in combination with other useful laboratory or clinical parameters is used for detecting the presence of a neurodegenerative disease or of a neurodegenerative disease stage in a patient liable to be afflicted by a neurodegenerative disease. Thus it allows determining either the presence of a neurodegenerative disease at an early stage or to discriminate between several stages of the pathology in order to evaluate the degree of severity of the disease.

As an example, in Alzheimer's disease, the Mild Cognitive Impairment (MCI), or light cognitive trouble, is proposed to define cognitive stage lesser than the one for an healthy people at the same age and socio cultural level, but nevertheless not serious enough to establish a dementia diagnostic.

MCI patients have nevertheless a major risk to further develop a heavy form such as dementia.

The expression ".....for the prognostic of a neurodegenerative disease or of a neurodegenerative disease stage in a patient liable to be afflicted by a neurodegenerative disease" means that the determination of the level of the secreted, extra cellular form of the soluble part of the (pro)renin receptor as set forth by SEQ ID NO:1 or fragments thereof, either alone or in conjunction with other useful laboratory or clinical parameters is used for predicting the risk of a patient or for identifying a patient having an enhanced risk for contracting a neurodegenerative disease or of a neurodegenerative disease stage.

In the present invention the term "prediction" denotes a prognosis of how a subject's (e.g. a patient's) medical condition will progress. This may include an estimation of the chance of recovery or the chance of an adverse outcome for said subject, e.g. a tumoral and/or proliferative disorder.

Said diagnosis or prognosis can be carried out by a method which may be selected from the following alternatives:
- comparison with the median of the level of the secreted, extra cellular form of the soluble part of the (pro)renin receptor as set forth by SEQ ID NO:1 or fragments thereof, in a set of pre-determined samples in a population of apparently healthy subjects,
- comparison with a quantile of the level of the secreted, extra cellular form of the soluble part of the (pro)renin receptor as set forth by SEQ ID NO:1 or fragments thereof, in a set of pre-determined samples in a population of apparently healthy subjects,
- calculation based on Cox Proportional Hazards analysis or by using risk index calculations such as the NRI (Net Reclassification Index) or the IDI (Integrated Discrimination Index); The index being liable to be calculated according to Pencina (Pencina MJ, et al.: Evaluating the added predictive ability of a new marker: from area under the ROC curve to reclassification and beyond. Stat Med. 2008;27:157-172).

The expression "for its use in the follow up of a treatment against a neurodegenerative disease in a patient afflicted by a neurodegenerative disease" means that the determination of the level of the secreted, extra cellular form of the soluble part of the (pro)renin receptor as set forth by SEQ ID NO:1 or fragments thereof, determined as above, allows measuring the efficacy of a treatment against a neurodegenerative disease,
either by coming back to normal values (for instance the median in an ensemble of pre-determined samples in a population of apparently healthy subjects) of said level of secreted, extra cellular form of the soluble part of the (pro)renin receptor as set forth by SEQ ID NO:1 or fragments thereof, indicating that the disease has been cured, or
by a decrease or increase of said level of secreted, extra cellular form of the soluble part of the (pro)renin receptor as set forth by SEQ ID NO:1 or fragments thereof,
compared with the one before treatment indicating respectively that:
the disease evolves through a lesser advanced stage or even the cure, showing that the treatment must be followed, or
the disease evolves through a more advanced stage, showing that the treatment is non effective and must be changed.
The expression "for its use in the follow up of a treatment stimulating the neuroregeneration" means that the determination of the level of the secreted, extra cellular form of the soluble part of the (pro)renin receptor as set forth by SEQ ID NO:1 or fragments thereof, determined as above, allows measuring the efficacy of a treatment against a neurodegenerative disease,
by a decrease or increase of said level of secreted, extra cellular form of the soluble part of the (pro)renin receptor as set forth by SEQ ID NO:1 or fragments thereof,
compared with the one before treatment indicating respectively that:
the neuroregeneration is increased, showing that the treatment must be followed, or
the neuroregeneration is decreased, showing that the treatment is non effective and must be changed.

The expression "for its use in the screening of new drugs liable to treat neurodegenerative pathologies" has the same meaning as the expression "for the follow up of a treatment against a neurodegenerative disease in a patient afflicted by a neurodegenerative disease" except that a new drug liable to treat neurodegenerative pathologies is used instead of a known treatment.

The expressions "for its use in the diagnosis ...of tumoral and/or proliferative disorders of the central nervous system, in particular glioblastomas" and "...for its use in the prognostic of tumoral and/or proliferative disorders of the central nervous system, in particular glioblastomas" have the same meaning as respectively the expression "for its use in the diagnosis ... of a neurodegenerative disease or of a neurodegenerative disease stage in a patient liable to be afflicted by a neurodegenerative disease" "... for its use in the prognostic of a neurodegenerative disease or of a neurodegenerative disease stage in a patient liable to be afflicted by a neurodegenerative disease", except that pathologies are tumoral and/or proliferative disorders of the central nervous system, in particular glioblastomas and not a neurodegenerative disease. Nevertheless, tumoral and/or proliferative disorders of the central nervous system give also a neurodegeneration.

The expression "for its use in the follow-up of tumors of the central nervous system after surgery and/or chemotherapy" means that the determination of the level of the secreted, extra cellular form of the soluble part of the (pro)renin receptor as set forth by SEQ ID NO:1 or fragments thereof, determined as above, allows measuring the efficacy of said surgery and/or chemotherapy,
by an increase or decrease of said level of secreted, extra cellular form of the soluble part of the (pro)renin receptor as set forth by SEQ ID NO:1 or fragments thereof,
compared with the one before said surgery and/or chemotherapy indicating respectively that:
said surgery and/or chemotherapy is effective, showing in the case of chemotherapy that the treatment must be followed, or
said surgery and/or chemotherapy is non effective, showing that the tumor has evolved and/or that chemotherapy is non effective and must be changed.

By "tumoral and/or proliferative disorders of the central nervous system" is meant the pathologies selected from the list consisting of glioblastomas and gangliogliomas.

The present invention relates to the use of the secreted, extra cellular form of the soluble part of the (pro)renin receptor as set forth by SEQ ID NO:1 or fragments thereof, the level of which has been determined *in vitro* in a sample of a biological fluid previously collected in a patient or an animal,
for the diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis,
for the follow up of a treatment against a neurodegenerative disease in a patient afflicted by a neurodegenerative disease, in particular Alzheimer's disease, dementia with Lewy bodies, fronto-temporal dementia, Parkinson's disease, amyotrophic lateral sclerosis, and/or
for the follow up of a treatment stimulating the neuroregeneration, and/or
for the screening of new drugs liable to treat neurodegenerative pathologies, in particular Alzheimer's disease, dementia with Lewy bodies, fronto-temporal dementia, Parkinson's disease, amyotrophic lateral sclerosis, and/or
for the diagnosis and/or the prognosis of tumoral and/or proliferative disorders of the central nervous system, in particular glioblastomas, and /or
for the follow-up of tumors of the central nervous system after surgery and/or chemotherapy, said level being modulated with respect to a sample of said biological fluid previously collected in a control patient or a control animal. Disclosed are the secreted, extra cellular form of the soluble part of the (pro)renin receptor as set forth by SEQ ID NO:1 or fragments thereof, the level of which has been determined *in vitro* in a sample of a biological fluid previously collected in a patient or an animal,
for its use in the diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis,
for its use in the follow up of a treatment against a neurodegenerative disease in a patient afflicted by a neurodegenerative disease, and/or
for its use in the follow up of a treatment stimulating the neuroregeneration, and/or
for its use in the screening of new drugs liable to treat neurodegenerative pathologies, and/or
for its use in the diagnosis and/or the prognosis of tumoral and/or proliferative disorders of the central nervous system, in particular glioblastomas, and /or
for its use in the follow-up of a treatment against tumoral and/or proliferative disorders of the central nervous system, in particular glioblastomas, after surgery and/or chemotherapy and/or radiotherapy,
wherein the level of the secreted, extra cellular form of the soluble part of the (pro)renin receptor as set forth by SEQ ID NO:1 or fragments thereof, under or over a certain threshold is indicative for:
the disease, or
the neuroregeneration, or
the efficacy of a treatment,
the discovery of a new drug, or
the efficacy of a surgery and/or a chemotherapy.

The skilled person can calculate these thresholds using standard statistical methods, e.g. Receiver Operating Characteristic analysis (ROC analysis) or by determining the median or mean or a desired percentile.

The invention may also involve comparing the level or concentration of a marker (here sPRR or fragments thereof) for the individual with a predetermined value. The predetermined value can take a variety of forms. It can be single cut-off value, such as for instance a median or mean or the 75th, 90th, 95th or 99th percentile of a population. It can be established based upon comparative groups, such as where the risk in one defined group is double the risk in another defined group. It can be a range, for example, where the tested population is divided equally (or unequally) into groups, such as a low-risk group, a medium-risk group and a high-risk group, or into quartiles, the lowest quartile being individuals with the lowest risk and the highest quartile being individuals with the highest risk.

The predetermined value can vary among particular populations selected, depending on their habits, ethnicity, genetics etc.

The present invention also relates to the use of the secreted, extra cellular form of the soluble part of the (pro)renin receptor as set forth by SEQ ID NO:1 or fragments thereof, the level of which has been determined *in vitro* in a sample of a biological fluid previously collected in a patient or an animal,
for the diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis,
for the follow up of a treatment against a neurodegenerative disease in a patient afflicted by a neurodegenerative disease, and/or
for the follow up of a treatment stimulating the neuroregeneration, and/or
for the screening of new drugs liable to treat neurodegenerative pathologies, and/or
for the diagnosis and/or the prognosis of tumoral and/or proliferative disorders of the central nervous system, in particular glioblastomas, and /or
for the follow-up of a treatment against tumoral and/or proliferative disorders of the central nervous system, in particular glioblastomas, after surgery and/or chemotherapy and/or radiotherapy,
wherein the level of the secreted, extra cellular form of the soluble part of the (pro)renin receptor as set forth by SEQ ID NO:1 or fragments thereof, under or over a certain threshold is indicative for:
the disease, or
the neuroregeneration, or
the efficacy of a treatment,
the discovery of a new drug, or
the efficacy of a surgery and/or a chemotherapy. Disclosed are the secreted, extra cellular form of the soluble part of the (pro)renin receptor as set forth by SEQ ID NO:1 or fragments thereof, the level of which is determined in a biological fluid of a patient or an animal, for its use in the diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis,
for its use in the follow up of a treatment against a neurodegenerative disease in a patient afflicted by a neurodegenerative disease, and/or
for its use in the follow up of a treatment stimulating the neuroregeneration, and/or
for its use in the screening of new drugs liable to treat neurodegenerative pathologies, and/or
for its use in the diagnosis and/or the prognosis of tumoral and/or proliferative disorders of the central nervous system, in particular glioblastomas, and /or
for its use in the follow-up of a treatment against tumoral and/or proliferative disorders of the central nervous system, in particular glioblastomas, after surgery and/or chemotherapy and/or radiotherapy,
said level being modulated with respect to a sample of said biological fluid of a control patient or a control animal.

Thus, in this embodiment, the use of SEQ ID NO: 1 is carried out *ex vivo.*

The present invention also relates to the use of the secreted, extra cellular form of the soluble part of the (pro)renin receptor as set forth by SEQ ID NO:1 or fragments thereof, the level of which is determined in a biological fluid of a patient or an animal,
for the diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis,
for the follow up of a treatment against a neurodegenerative disease in a patient afflicted by a neurodegenerative disease, and/or
for the follow up of a treatment stimulating the neuroregeneration, and/or
for the screening of new drugs liable to treat neurodegenerative pathologies, and/or
for the diagnosis and/or the prognosis of tumoral and/or proliferative disorders of the central nervous system, in particular glioblastomas, and /or
for the follow-up of a treatment against tumoral and/or proliferative disorders of the central nervous system, in particular glioblastomas, after surgery and/or chemotherapy and/or radiotherapy,
said level being modulated with respect to a sample of said biological fluid of a control patient or a control animal. Disclosed are the secreted, extra cellular form of the soluble part of the (pro)renin receptor as set forth by SEQ ID NO:1 or fragments thereof, the level of which has been determined in a biological fluid previously collected in a patient or an animal, as defined above,
for its use in the diagnosis and/or the prognostic of a neurodegenerative disease or of a neurodegenerative disease stage, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than the one as determined in control patients, in particular higher than 42 ng/mL, in particular higher than 50 ng/ml, as determined in control patients.

42 ng/ml represents a mean value as determined on controls with the Elisa test from IBL (code number 27782).

The test Elisa is presented in the Examples.

The inventors have found that a level of the secreted, extra cellular form of the soluble part of the (pro)renin receptor as set forth by SEQ ID NO:1 above about 42 ng/ml, in particular above about 50 ng/mL, in the cerebrospinal fluid (CSF), may be indicative of either an early stage of a neurodegenerative disease or of an advanced stage, or a late stage of neurodegenerative disease, in particular Alzheimer's disease.

sPRR can also be used as biomarker of dementia with Lewy bodies, fronto-temporal dementia, Parkinson's disease, amyotrophic lateral sclerosis or of a risk to develop an neurodegenerative disease.

50 ng/ml is a threshold giving a sensitivity and specificity of 75% (15 patients/20 controls and AD patients, i.e. 15 controls/20 are below this value and 15 patients/20 are above this value.

Nevertheless, said level of about 50 ng/mL is a threshold value but the genetic background of an individual may lead to higher or lower value also in a healthy human. Therefore, the following value is preferably plus/minus 50%, plus/minus 40%, plus/minus 30%, plus/minus 25%, plus/minus 20%, plus/minus 15%, plus/minus 10%, plus/minus 5% or plus/minus 2.5%.

The present invention also relates to the use of the secreted, extra cellular form of the soluble part of the (pro)renin receptor as set forth by SEQ ID NO:1 or fragments thereof, the level of which has been determined in a biological fluid previously collected in a patient or an animal, as defined above,
for the diagnosis and/or the prognostic of a neurodegenerative disease or of a neurodegenerative disease stage, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than the one as determined in control patients, in particular higher than 42 ng/mL, in particular higher than 50 ng/ml, as determined in control patients.

In an advantageous embodiment, the level of the secreted, extra cellular form of the soluble part of the (pro)renin receptor as set forth by SEQ ID NO:1 for a patient afflicted by a MCI is comprised from about 50 ng/mL to about 200ng/mL, preferentially from about 50 ng/mL to about 100 ng/mL, preferentially from about 60 ng/mL to about 100 ng/mL, preferentially from about 70 ng/mL to about 100 ng/mL, preferentially from about 70 ng/mL to about 90 ng/mL, in particular 80 ng/mL the values increasing with the severity of the cognitive impairment.

In an advantageous embodiment, the level of the secreted, extra cellular form of the soluble part of the (pro)renin receptor as set forth by SEQ ID NO:1 for a patient afflicted by a heavy stage of Alzheimer's disease is higher than the one for a patient afflicted by a MCI. Disclosed are the secreted, extra cellular form of the soluble part of the (pro)renin receptor as set forth by SEQ ID NO:1 or fragments thereof, the level of which is determined in a biological fluid of a patient or an animal, as defined above,
for its use in the diagnosis and/or the prognostic of a neurodegenerative disease or of a neurodegenerative disease stage, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than about 50 ng/mL.

Thus, in this embodiment, the use of SEQ ID NO:1 is carried out *ex vivo.*

The present invention also relates to the use of the secreted, extra cellular form of the soluble part of the (pro)renin receptor as set forth by SEQ ID NO:1 or fragments thereof, the level of which is determined in a biological fluid of a patient or an animal, as defined above,
for the diagnosis and/or the prognostic of a neurodegenerative disease or of a neurodegenerative disease stage, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than about 50 ng/mL. Disclosed are the secreted, extra cellular form of the soluble part of the (pro)renin receptor as set forth by SEQ ID NO:1 or fragments thereof, the level of which has been determined in a biological fluid previously collected in a patient or an animal, as defined above,
for its use in the follow up of a treatment against a neurodegenerative disease in a patient or animal, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal previously treated with said treatment, is lower than the one determined in said patient or animal before said treatment.

In this embodiment, it is clear that said follow up is carried out *in vitro* after treatment; said treatment is not included in the method or process of follow up.

A patient or animal that has been diagnosed with a neurodegenerative disease, having thus a level of the secreted, extra cellular form of the soluble part of the (pro)renin receptor as set forth by SEQ ID NO:1, in the CSF, higher than about 50 ng/mL, must be treated with a drug against a neurodegenerative disease. After several days, weeks or months of treatments, depending on the treatment, an evaluation of the efficacy of said drug or treatment must be done (follow up).

A decrease of the level of the secreted, extra cellular form of the soluble part of the (pro)renin receptor as set forth by SEQ ID NO:1, in CSF, compared with the one obtained before said treatment, is indicative of the efficacy of said drug or treatment while a stability, an increase of said level is indicative of the inefficacy of the drug that must therefore be changed.

The present invention also relates to the use of the secreted, extra cellular form of the soluble part of the (pro)renin receptor as set forth by SEQ ID NO:1 or fragments thereof, the level of which has been determined in a biological fluid previously collected in a patient or an animal, as defined above,
for the follow up of a treatment against a neurodegenerative disease in a patient or animal, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal previously treated with said treatment, is lower than the one determined in said patient or animal before said treatment. Disclosed are the secreted, extra cellular form of the soluble part of the (pro)renin receptor as set forth by SEQ ID NO:1 or fragments thereof, the level of which is determined in a biological fluid of a patient or an animal, as defined above,
for its use in the follow up of a treatment against a neurodegenerative disease in a patient or animal, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal treated with said treatment, is lower than the one determined in said patient or animal before said treatment.

Thus, in this embodiment, the use of SEQ ID NO:1 is carried out *ex vivo.*

The present invention also relates to the use of the secreted, extra cellular form of the soluble part of the (pro)renin receptor as set forth by SEQ ID NO:1 or fragments thereof, the level of which is determined in a biological fluid of a patient or an animal, as defined above,
for the follow up of a treatment against a neurodegenerative disease in a patient or animal, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal treated with said treatment, is lower than the one determined in said patient or animal before said treatment.

In an advantageous embodiment the present invention relates to the secreted, extra cellular form of the soluble part of the (pro)renin receptor as set forth by SEQ ID NO:1 or fragments thereof, the level of which has been determined in a biological fluid previously collected in a patient or an animal, as defined above,
for its use in the follow up of a treatment stimulating the neuroregeneration in a patient or animal, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal previously treated with said treatment, is lower than about 50 ng/mL.

In this embodiment, it is clear that said follow up is carried out *in vitro* after treatment; said treatment is not included in the method or process of follow up.

A patient or animal necessitating a neuroregeneration treatment must have a follow up to evaluate the efficacy of the treatment after several days, weeks or months of treatments, depending on the treatment.

A decrease of the level of the secreted, extra cellular form of the soluble part of the (pro)renin receptor as set forth by SEQ ID NO:1, in CSF, compared with the one obtained before said treatment, is indicative of the efficacy of said drug or treatment while a stability or a decrease of less than 10% of said level is indicative of the inefficacy of the drug that must therefore be changed.

The present invention also relates to the use of the secreted, extra cellular form of the soluble part of the (pro)renin receptor as set forth by SEQ ID NO:1 or fragments thereof, the level of which has been determined in a biological fluid previously collected in a patient or an animal, as defined above,
for its use in the follow up of a treatment stimulating the neuroregeneration in a patient or animal, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal previously treated with said treatment, is lower than about 50 ng/mL. Disclosed are the secreted, extra cellular form of the soluble part of the (pro)renin receptor as set forth by SEQ ID NO:1 or fragments thereof, the level of which is determined in a biological fluid of a patient or an animal, as defined above,
for its use in the follow up of a treatment stimulating the neuroregeneration in a patient or animal, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient treated with said treatment, is lower than about 50 ng/mL.

Thus, in this embodiment, the use of SEQ ID NO:1 is carried out *ex vivo.*

The present invention also relates to the use of the secreted, extra cellular form of the soluble part of the (pro)renin receptor as set forth by SEQ ID NO:1 or fragments thereof, the level of which is determined in a biological fluid of a patient or an animal, as defined above,
for the follow up of a treatment stimulating the neuroregeneration in a patient or animal, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient treated with said treatment, is lower than about 50 ng/mL. Disclosed are the secreted, extra cellular form of the soluble part of the (pro)renin receptor as set forth by SEQ ID NO:1 or fragments thereof, the level of which has been determined in a biological fluid previously collected in a patient or an animal, as defined above,
for its use in the screening of new drugs liable to treat neurodegenerative pathologies in a patient or animal, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal afflicted by a neurodegenerative disease, previously treated with said new drug, is lower than the one determined in said patient or animal before the treatment with said new drug, or equal to the one determined in control patients.

In this embodiment, it is clear that said screening of new drugs is carried out *in vitro* after treatment with said drug, said treatment is thus not included in the method or process of follow up.

A decrease of the level of the secreted, extra cellular form of the soluble part of the (pro)renin receptor as set forth by SEQ ID NO:1, in CSF, compared with the one obtained before said treatment, or a level of the secreted, extra cellular form of the soluble part of the (pro)renin receptor as set forth by SEQ ID NO:1, in CSF, substantially equivalent with the one obtained in control patients or animals, is indicative of the efficacy of said new drug.

The present invention also relates to the use of the secreted, extra cellular form of the soluble part of the (pro)renin receptor as set forth by SEQ ID NO:1 or fragments thereof, the level of which has been determined in a biological fluid previously collected in a patient or an animal, as defined above,
for the screening of new drugs liable to treat neurodegenerative pathologies in a patient or animal, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal afflicted by a neurodegenerative disease, previously treated with said new drug, is lower than the one determined in said patient or animal before the treatment with said new drug, or equal to the one determined in control patients. Disclosed are the secreted, extra cellular form of the soluble part of the (pro)renin receptor as set forth by SEQ ID NO:1 or fragments thereof, the level of which is determined in a biological fluid of a patient or an animal, as defined above,
for its use in the screening of new drugs liable to treat neurodegenerative pathologies in a patient or animal, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal afflicted by a neurodegenerative disease, previously treated with said new drug, is lower than the one determined in said patient or animal before the treatment with said new drug, or equal to the one determined in control patients.

Thus, in this embodiment, the use of SEQ ID NO:1 is carried out *ex vivo.*

The present invention also relates to the use of the secreted, extra cellular form of the soluble part of the (pro)renin receptor as set forth by SEQ ID NO:1 or fragments thereof, the level of which is determined in a biological fluid of a patient or an animal, as defined above,
for the screening of new drugs liable to treat neurodegenerative pathologies in a patient or animal, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient or animal afflicted by a neurodegenerative disease, previously treated with said new drug, is lower than the one determined in said patient or animal before the treatment with said new drug, or equal to the one determined in control patients. Disclosed are the secreted, extra cellular form of the soluble part of the (pro)renin receptor as set forth by SEQ ID NO:1 or fragments thereof, the level of which has been determined in a biological fluid previously collected in a patient or an animal, as defined above,
for its use in the diagnosis and/or the prognosis of tumoral and/or proliferative disorders of the central nervous system, in particular glioblastomas, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than about 42 ng/mL, in particular higher than about 50 ng/ml, as determined in control patients.

The inventors have found that a level of the secreted, extra cellular form of the soluble part of the (pro)renin receptor as set forth by SEQ ID NO:1 above about 42 ng/mL, in the cerebrospinal fluid (CSF), may be indicative of tumoral and/or proliferative disorders of the central nervous system, in particular glioblastomas.

Nevertheless, said level of about 42 ng/mL is a threshold value but the genetic background of an individual may lead to higher or lower value also in an healthy human or animal. Therefore, the following value is preferably plus/minus 50%, plus/minus 40%, plus/minus 30%, plus/minus 25%, plus/minus 20%, plus/minus 15%, plus/minus 10%, plus/minus 5% or plus/minus 2.5%.

The present invention also relates to the use of the secreted, extra cellular form of the soluble part of the (pro)renin receptor as set forth by SEQ ID NO:1 or fragments thereof, the level of which has been determined in a biological fluid previously collected in a patient or an animal, as defined above,
for its use in the diagnosis and/or the prognosis of tumoral and/or proliferative disorders of the central nervous system, in particular glioblastomas, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than about 42 ng/mL, in particular higher than about 50 ng/ml, as determined in control patients. Disclosed are the secreted, extra cellular form of the soluble part of the (pro)renin receptor as set forth by SEQ ID NO:1 or fragments thereof, the level of which is determined in a biological fluid of a patient or an animal, as defined above,
for its use in the diagnosis and/or the prognosis of tumoral and/or proliferative disorders of the central nervous system, in particular glioblastomas, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than about 42 ng/mL, in particular 50 ng/ml, as determined in control patients.

Thus, in this embodiment, the use of SEQ ID NO:1 is carried out *ex vivo.*

The present invention also relates to the use of the secreted, extra cellular form of the soluble part of the (pro)renin receptor as set forth by SEQ ID NO:1 or fragments thereof, the level of which is determined in a biological fluid of a patient or an animal, as defined above,
for the diagnosis and/or the prognosis of tumoral and/or proliferative disorders of the central nervous system, in particular glioblastomas, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than about 42 ng/mL, in particular 50 ng/ml, as determined in control patients. Disclosed are the secreted, extra cellular form of the soluble part of the (pro)renin receptor as set forth by SEQ ID NO:1 or fragments thereof, the level of which has been determined in a biological fluid previously collected in a patient or an animal, as defined above,
for its use in the follow-up of tumors of the central nervous system after surgery and/or chemotherapy, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient subjected to a surgery and/or chemotherapy is lower than the one determined in said patient or animal before surgery and/or chemotherapy, or equal to the one determined in control patients.

In this embodiment, it is clear that said followed up is carried out *in vitro* after surgery and/or chemotherapy; said surgery and/or chemotherapy is not included in the method or process of follow up.

A patient or animal that has been subjected to a surgery and/or chemotherapy for tumors of the central nervous system, having thus a level of the secreted, extra cellular form of the soluble part of the (pro)renin receptor as set forth by SEQ ID NO:1, in the CSF, above about 42 ng/mL, in particular 50 ng/ml, before said surgery and/or chemotherapy, must be follow up for evaluation the efficacy of the surgery and/or chemotherapy. After several days, weeks or months of surgery and/or chemotherapy and/or radiotherapy, depending on the chemotherapy and/or the radiotherapy, an evaluation of the efficacy of said surgery and/or chemotherapy and/or radiotherapy must be done (follow up).

A decrease of the level of the secreted, extra cellular form of the soluble part of the (pro)renin receptor as set forth by SEQ ID NO:1, in CSF, compared with the one obtained before said surgery and/or chemotherapy, is indicative of the efficacy of said surgery and/or chemotherapy while a stability or an increase of said level is indicative of the inefficacy of the surgery and/or chemotherapy and/or radiotherapy.

The present invention also relates to the use of the secreted, extra cellular form of the soluble part of the (pro)renin receptor as set forth by SEQ ID NO:1 or fragments thereof, the level of which has been determined in a biological fluid previously collected in a patient or an animal, as defined above,
for the follow-up of tumors of the central nervous system after surgery and/or chemotherapy, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient subjected to a surgery and/or chemotherapy is lower than the one determined in said patient or animal before surgery and/or chemotherapy, or equal to the one determined in control patients. Disclosed are the secreted, extra cellular form of the soluble part of the (pro)renin receptor as set forth by SEQ ID NO:1 or fragments thereof, the level of which is determined in a biological fluid of in a patient or an animal, as defined above,
for its use in the follow-up of tumors of the central nervous system after surgery and/or chemotherapy, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient subjected to a surgery and/or chemotherapy is lower than the one determined in said patient or animal before surgery and/or chemotherapy, or equal to the one determined in control patients.

Thus, in this embodiment, the use of SEQ ID NO:1 is carried out *ex vivo.*

The present invention also relates to the use of the secreted, extra cellular form of the soluble part of the (pro)renin receptor as set forth by SEQ ID NO:1 or fragments thereof, the level of which is determined in a biological fluid of in a patient or an animal, as defined above,
for its use in the follow-up of tumors of the central nervous system after surgery and/or chemotherapy, wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient subjected to a surgery and/or chemotherapy is lower than the one determined in said patient or animal before surgery and/or chemotherapy, or equal to the one determined in control patients.

In another aspect, the present invention relates to an *in vitro* process
for the diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis, and/or
for the follow up of a treatment against a neurodegenerative disease in a patient afflicted by a neurodegenerative disease, in particular Alzheimer's disease, dementia with Lewy bodies, fronto-temporal dementia, Parkinson's disease, amyotrophic lateral sclerosis, and/or
for the follow up of a treatment stimulating the neuroregeneration, and/or
for the screening of new drugs liable to treat neurodegenerative pathologies, in particular Alzheimer's disease, dementia with Lewy bodies, fronto-temporal dementia, Parkinson's disease, amyotrophic lateral sclerosis, and/or
for the diagnosis and/or the prognosis of tumoral and/or proliferative disorders of the central nervous system, in particular glioblastomas, and /or
for the follow-up of a treatment against tumoral and/or proliferative disorders of the central nervous system, in particular glioblastomas, after surgery and/or chemotherapy and/or radiotherapy,
comprising the *in vitro* determination of the level of the secreted, extra cellular form of the soluble part of the (pro)renin receptor as set forth by SEQ ID NO:1 or fragments thereof, in a sample of a biological fluid previously collected in a patient or an animal.

Said process is carried out after collecting a sample of a biological fluid in a patient or animal.

If the patient or animal is treated with a drug against a neurodegenerative disease, or a drug stimulating the neuroregeneration or new drugs liable to treat neurodegenerative pathologies or has been subjected to a surgery and/or chemotherapy and/or radiotherapy, all these treatments, surgery, chemotherapy or radiotherapy are carried out previously to said process of the invention.

Thus said process is liable to be used as a diagnostic and/or prognostic tool for neurodegenerative diseases, either at an early stage or to discriminate between several stages of the pathology in order to evaluate the degree of severity of the disease, or tumoral and/or proliferative disorders as well as a follow up tool of the efficacy of a treatment against neurodegenerative diseases or tumoral and/or proliferative disorders or for the stimulation of the neuroregeneration, as well as a tool to discover new drugs liable to treat neurodegenerative pathologies.

The determination of the level of the secreted, extra cellular form of the soluble part of the (pro)renin receptor as set forth by SEQ ID NO:1 or fragments thereof, can be carried out with an Elisa test using antibodies obtained from the epitopes of SEQ ID NO:1 as set forth by SEQ ID NO:4-8 with techniques well known from a man skilled in the art.

A threshold value can be determined for each biological fluid in control patients or animals with said Elisa test.

In function of the pathologies the level of the secreted, extra cellular form of the soluble part of the (pro)renin receptor as set forth by SEQ ID NO:1, or fragments thereof, will be higher or lower than the threshold value.

Nevertheless, the genetic background of an individual may lead to higher or lower value also in a healthy human or animal. Therefore, the value may preferably plus/minus 50%, plus/minus 40%, plus/minus 30%, plus/minus 25%, plus/minus 20%, plus/minus 15%, plus/minus 10%, plus/minus 5% or plus/minus 2.5% than the determined threshold value.

In another aspect, the present invention relates to a process
for the diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis, and/or
for the follow up of a treatment against a neurodegenerative disease in a patient afflicted by a neurodegenerative disease, in particular Alzheimer's disease, dementia with Lewy bodies, fronto-temporal dementia, Parkinson's disease, amyotrophic lateral sclerosis, and/or
for the follow up of a treatment stimulating the neuroregeneration, and/or
for the screening of new drugs liable to treat neurodegenerative pathologies, in particular Alzheimer's disease, dementia with Lewy bodies, fronto-temporal dementia, Parkinson's disease, amyotrophic lateral sclerosis, and/or
for the diagnosis and/or the prognosis of tumoral and/or proliferative disorders of the central nervous system, in particular glioblastomas, and /or
for the follow-up of a treatment against tumoral and/or proliferative disorders of the central nervous system, in particular glioblastomas, after surgery and/or chemotherapy and/or radiotherapy,
comprising the determination of the level of the secreted, extra cellular form of the soluble part of the (pro)renin receptor as set forth by SEQ ID NO:1 or fragments thereof, in a biological fluid of a patient or an animal.

Thus in this embodiment, said process is carried out *ex vivo* and said process comprises the treatment with the drug against a neurodegenerative disease, the drug stimulating the neuroregeneration or with new drugs liable to treat neurodegenerative pathologies or with the surgery and/or chemotherapy and/or radiotherapy.

It also comprises the collecting of a biological fluid in a patient or animal.

In an advantageous embodiment, the present invention relates to an *in vitro* process defined above,
for the diagnosis and/or the prognostic of a neurodegenerative disease in a patient liable to be afflicted by a neurodegenerative disease, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid,
comprising the following steps:
a. *In vitro* determination of the level of the secreted, extra cellular form of the soluble part of the (pro)renin receptor as set forth by SEQ ID NO:1 or fragments thereof, as determined by an Elisa test, in a patient liable to be afflicted by a neurodegenerative disease;
b. *In vitro* determination of the level of secreted, extra cellular form of the soluble part of the (pro)renin receptor as set forth by SEQ ID NO:1 or fragments thereof, as determined by an Elisa test, in control patients or animals;
c. Comparison of both values, the value determined in step a. being higher than the one of step b. being indicative of a neurodegenerative disease or a risk to have a neurodegenerative disease.

In this embodiment, said process is carried out after collecting a sample of a biological fluid in a patient or animal.

The level of secreted, extra cellular form of the soluble part of the (pro)renin receptor as set forth by SEQ ID NO:1 or fragments thereof, as determined by an Elisa test, in control patients is in the range of (or about) 42 ng/mL in CSF.

The Inventors have found that in the case of neurodegenerative diseases, the level of secreted, extra cellular form of the soluble part of the (pro)renin receptor as set forth by SEQ ID NO:1 or fragments thereof in patients or animal afflicted by a neurodegenerative disease (as determined in step a) is higher than 50 ng/mL.

Thus in function of the determined value, a patient will be submitted to a diagnostic or prognostic to have a neurodegenerative disease, in particular an early diagnostic or prognostic to have a neurodegenerative disease. If such value is about equal to the one of the control patients or animal, it could be concluded that the patient is not afflicted by a neurodegenerative disease.

In an advantageous embodiment, the level of the secreted, extra cellular form of the soluble part of the (pro)renin receptor as set forth by SEQ ID NO:1 for a patient afflicted by a heavy stage of Alzheimer's disease is higher than about 50 ng/mL, in particular from 50 ng/ml to 200 ng/ml.

Thus, the process of the invention allows also evaluating the degree of severity of the neurodegenerative pathology.

The process of the invention also allows to follow-up the progression of the disease and to be used as a prognostic biomarker of the progression of the disease and of the evolution of Alzheimer's disease and the progression of MCI to Alzheimer's disease.

In an advantageous embodiment, the present invention relates to a process defined above, for the diagnosis and/or the prognostic of a neurodegenerative disease in a patient liable to be afflicted by a neurodegenerative disease, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid,
comprising the following steps:
a. Determination of the level of the secreted, extra cellular form of the soluble part of the (pro)renin receptor as set forth by SEQ ID NO:1 or fragments thereof, as determined by an Elisa test, in a patient liable to be afflicted by a neurodegenerative disease;
b. Determination of the level of secreted, extra cellular form of the soluble part of the (pro)renin receptor as set forth by SEQ ID NO:1 or fragments thereof, as determined by an Elisa test, in control patients or animals;
c. Comparison of both values, the value determined in step a. being higher than the one of step b. being indicative of a neurodegenerative disease or a risk to have a neurodegenerative disease.

Thus in this embodiment, said process is carried out *ex vivo.*

In an advantageous embodiment, the present invention relates to an *in vitro* process defined above,
for the follow up of a treatment against a neurodegenerative disease, wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO:1 or fragments thereof, as determined by an Elisa test, in a patient or animal previously treated with said treatment,
b. *In vitro* determination of the level of the secreted, extra cellular form of the soluble part of the (pro)renin receptor as set forth by SEQ ID NO:1 or fragments thereof, as determined by an Elisa test, in said patient before said treatment;
c. Comparison of both values, the value determined in step a. being lower than the one of step b. being indicative of the efficacy of said treatment.

In this embodiment, said process is carried out after collecting a sample of a biological fluid in a patient or animal.

The treatment is carried out previously to said process of the invention.

In the case of a patient or animal that is afflicted by a neurodegenerative disease, the efficacy of the treatment will be evaluated by the decrease of the level of the secreted, extra cellular form of the soluble part of the (pro)renin receptor as set forth by SEQ ID NO:1 or fragments thereof compared with control patients or animals.

In the case where said level determined in step a is comprised between the value determined in step b) and higher than 50 ng/mL, it can be concluded that the treatment has an efficacy but must be continued.

In the case where said level determined in step a is below 50 ng/ml, in particular about 42 ng/mL, it can be concluded that the treatment has an efficacy and the patient has been cured.

In the case where said level determined in step a is about the value determined in step b) or higher than it, it can be concluded that the treatment has no efficacy and must be changed.

In an advantageous embodiment, the present invention relates to a process defined above, for the follow up of a treatment against a neurodegenerative disease, wherein said fluid is the cerebrospinal fluid, in particular the human cerebrospinal fluid, comprising the following steps:
a. Determination of the level of the secreted, extra cellular form of the soluble part of the (pro)renin receptor as set forth by SEQ ID NO:1 or fragments thereof, as determined by an Elisa test, in a patient or animal previously treated with said treatment,
b. Determination of the level of the secreted, extra cellular form of the soluble part of the (pro)renin receptor as set forth by SEQ ID NO:1 or fragments thereof, as determined by an Elisa test, in said patient before said treatment;
c. Comparison of both values, the value determined in step a. being lower than the one of step b. being indicative of the efficacy of said treatment.

Thus in this embodiment, said process is carried out *ex vivo.*

In an advantageous embodiment, the present invention relates to an *in vitro* process defined above,
for the follow up of a treatment stimulating the neuroregeneration in a previously treated patient,
wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO:1 or fragments thereof, as determined by an Elisa test, in a patient or animal previously treated with said treatment,
b. *In vitro* determination of the level of the secreted, extra cellular form of the soluble part of the (pro)renin receptor as set forth by SEQ ID NO:1 or fragments thereof, as determined by an Elisa test, in control patients or animals;
c. Comparison of both values, the value determined in step a. being lower than the one of step b. being indicative of the neuroregeneration.

In this embodiment, said process is carried out after collecting a sample of a biological fluid in a patient or animal.

The treatment is carried out previously to said process of the invention.

In the case of a patient or animal treated for neuroregeneration, the efficacy of the treatment will be evaluated by the decrease of the level of the secreted, extra cellular form of the soluble part of the (pro)renin receptor as set forth by SEQ ID NO:1 or fragments thereof of the treated patient compared with the one in a control patient or animal.

In the case where said level determined in step a is lower than 50 ng/mL (threshold value), it can be concluded that the treatment has an efficacy. In function of the difference between both value, said treatment can be stopped or continued.

In the case where said level determined in step a is higher than 50 ng/mL, it can be concluded that the treatment has no efficacy and must be changed.

In an advantageous embodiment, the present invention relates to a process defined above,
for the follow up of a treatment stimulating the neuroregeneration in a previously treated patient,
wherein said fluid is the cerebrospinal fluid, in particular the human cerebrospinal fluid, comprising the following steps:
a. Determination of the level of the secreted, extra cellular form of the soluble part of the (pro)renin receptor as set forth by SEQ ID NO:1 or fragments thereof, as determined by an Elisa test, in a patient or animal previously treated with said treatment,
b. Determination of the level of the secreted, extra cellular form of the soluble part of the (pro)renin receptor as set forth by SEQ ID NO:1 or fragments thereof, as determined by an Elisa test, in control patients or animals;
c. Comparison of both values, the value determined in step a. being lower than the one of step b. being indicative of the neuroregeneration.

Thus in this embodiment, said process is carried out *ex vivo.*

In an advantageous embodiment, the present invention relates to an *in vitro* process defined above,
for the screening of new drugs liable to treat neurodegenerative pathologies in a patient or animal, in particular Alzheimer's disease, dementia with Lewy bodies, fronto-temporal dementia, Parkinson's disease, amyotrophic lateral sclerosis,
wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO:1 or fragments thereof, as determined by an Elisa test, in a patient or animal afflicted by a neurodegenerative disease and previously treated with said new drug,
b. *In vitro* determination of the level of the secreted, extra cellular form of the soluble part of the (pro)renin receptor as set forth by SEQ ID NO:1 or fragments thereof, as determined by an Elisa test, in a patient or animal afflicted by a neurodegenerative disease before treatment or in control patients or animals;
c. Comparison of both values, the value determined in step a. for said patient previously treated with said new drug being lower than the one determined in step b. or equal to the one of control patients or animals, being indicative of an efficacy of said new drug.

In this embodiment, said process is carried out after collecting a sample of a biological fluid in a patient or animal.

The treatment is carried out previously to said process of the invention.

In the case of a patient or animal that is afflicted by a neurodegenerative disease, the efficacy of the new treatment will be evaluated by the decrease of the level of the secreted, extra cellular form of the soluble part of the (pro)renin receptor as set forth by SEQ ID NO:1 or fragments thereof of the treated patient compared with the one of control patients or animals.

In the case where said level determined in step a is comprised between the value determined in step b) and higher than 50 ng/mL, it can be concluded that the new treatment has an efficacy but must be continued.

In the case where said level determined in step a is lower than 50 ng/ml, in particular about 42 ng/mL, it can be concluded that the new treatment has an efficacy and the patient has been cured.

In the case where said level determined in step a is about the value determined in step b) or higher than it, it can be concluded that the treatment has no efficacy and must be changed.

In an advantageous embodiment, the present invention relates to a process defined above,
for the screening of new drugs liable to treat neurodegenerative pathologies in a patient or animal, in particular Alzheimer's disease, dementia with Lewy bodies, fronto-temporal dementia, Parkinson's disease, amyotrophic lateral sclerosis,
wherein said fluid is the cerebrospinal fluid, in particular the human cerebrospinal fluid, comprising the following steps:
a. Determination of the level of the secreted, extra cellular form of the soluble part of the (pro)renin receptor as set forth by SEQ ID NO:1 or fragments thereof, as determined by an Elisa test, in a patient or animal afflicted by a neurodegenerative disease and previously treated with said new drug,
b. Determination of the level of the secreted, extra cellular form of the soluble part of the (pro)renin receptor as set forth by SEQ ID NO:1 or fragments thereof, as determined by an Elisa test, in a patient or animal afflicted by a neurodegenerative disease before treatment or in control patients or animals;
c. Comparison of both values, the value determined in step a. for said patient previously treated with said new drug being lower than the one determined in step b. or equal to the one of control patients or animals, being indicative of an efficacy of said new drug.

Thus in this embodiment, said process is carried out *ex vivo.*

In an advantageous embodiment, the present invention relates to an *in vitro* process defined above,
for the diagnosis and/or the prognosis of tumoral and/or proliferative disorders of the central nervous system, in particular glioblastomas, wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO:1 or fragments thereof, as determined by an Elisa test, in a patient liable to be afflicted by tumoral and/or proliferative disorders of the central nervous system, in particular glioblastomas;
b. *In vitro* determination of the level of secreted, extra cellular form of the soluble part of the (pro)renin receptor as set forth by SEQ ID NO:1 or fragments thereof, as determined by an Elisa test, in control patients or animals;
c. Comparison of both values, the value determined in step a. being higher than the one of step b. being indicative of a tumoral and/or proliferative disorders of the central nervous system, in particular glioblastomas or a risk to have a tumoral and/or proliferative disorders of the central nervous system, in particular glioblastomas.

In this embodiment, said process is carried out after collecting a sample of a biological fluid in a patient or animal.

The Inventors have found that in the case of tumoral and/or proliferative disorders of the central nervous system, in particular glioblastomas, the level of secreted, extra cellular form of the soluble part of the (pro)renin receptor as set forth by SEQ ID NO:1 or fragments thereof in patients or animal afflicted by tumoral and/or proliferative disorders of the central nervous system, in particular glioblastomas, (as determined in step a) is higher than 42 ng/mL

In an advantageous embodiment, the present invention relates to a process defined above, for the diagnosis and/or the prognosis of tumoral and/or proliferative disorders of the central nervous system, in particular glioblastomas, wherein said fluid is the cerebrospinal fluid, in particular the human cerebrospinal fluid, comprising the following steps:
a. Determination of the level of the secreted, extra cellular form of the soluble part of the (pro)renin receptor as set forth by SEQ ID NO:1 or fragments thereof, as determined by an Elisa test, in a patient liable to be afflicted by tumoral and/or proliferative disorders of the central nervous system, in particular glioblastomas;
b. Determination of the level of secreted, extra cellular form of the soluble part of the (pro)renin receptor as set forth by SEQ ID NO:1 or fragments thereof, as determined by an Elisa test, in control patients or animals;
c. Comparison of both values, the value determined in step a. being higher than the one of step b. being indicative of a tumoral and/or proliferative disorders of the central nervous system, in particular glioblastomas or a risk to have a tumoral and/or proliferative disorders of the central nervous system, in particular glioblastomas.

Thus in this embodiment, said process is carried out *ex vivo.*

In an advantageous embodiment, the present invention relates to an *in vitro* process defined above,
for the follow-up of tumors of the central nervous system after surgery and/or chemotherapy and/or radiotherapy, wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO:1 or fragments thereof, as determined by an Elisa test, in a patient having a tumoral and/or proliferative disorders of the central nervous system, in particular glioblastomas and subjected to a surgery and/or chemotherapy and/or radiotherapy,
b. *In vitro* determination of the level of the secreted, extra cellular form of the soluble part of the (pro)renin receptor as set forth by SEQ ID NO:1 or fragments thereof, as determined by an Elisa test, in a patient having a tumoral and/or proliferative disorders of the central nervous system, in particular glioblastomas before said surgery and/or chemotherapy and/or radiotherapy,
c. Comparison of both values, the value determined in step a. being lower than the one of step b. being indicative of a the efficacy of surgery and/or chemotherapy and/or radiotherapy.

In this embodiment, said process is carried out after collecting a sample of a biological fluid in a patient or animal.

The surgery and/or chemotherapy and/or radiotherapy are carried out previously to said process of the invention.

In the case of a patient or animal that is afflicted by tumoral and/or proliferative disorders of the central nervous system, in particular glioblastomas, the level of the secreted, extra cellular form of the soluble part of the (pro)renin receptor as set forth by SEQ ID NO:1 or fragments thereof is higher than about 42 ng/mL.

The efficacy of the surgery and/or chemotherapy and/or radiotherapy will be thus evaluated by the decrease of the level of the secreted, extra cellular form of the soluble part of the (pro)renin receptor as set forth by SEQ ID NO:1 or fragments thereof in the patient with surgery and/or chemotherapy and/or radiotherapy compared with the one before surgery and/or chemotherapy and/or radiotherapy.

In the case where said level determined in step a is comprised between the value determined in step b) and higher than 42 ng/mL, it can be concluded that the surgery and/or chemotherapy and/or radiotherapy has an efficacy but chemotherapy and/or radiotherapy must be continued.

In the case where said level is about determined in step a is about 42 ng/mL, it can be concluded that the surgery and/or chemotherapy and/or radiotherapy has an efficacy and the patient has been cured.

In the case where said level determined in step a is about the value determined in step b) or less than it, it can be concluded that the surgery and/or chemotherapy and/or radiotherapy has no efficacy and and/or chemotherapy and/or radiotherapy must be changed or the patient must have another surgery.

In an advantageous embodiment, the present invention relates to a process defined above, for the follow-up of tumors of the central nervous system after surgery and/or chemotherapy and/or radiotherapy, wherein said fluid is the cerebrospinal fluid, in particular the human cerebrospinal fluid, comprising the following steps:
a. Determination of the level of the secreted, extra cellular form of the soluble part of the (pro)renin receptor as set forth by SEQ ID NO:1 or fragments thereof, as determined by an Elisa test, in a patient having a tumoral and/or proliferative disorders of the central nervous system, in particular glioblastomas and subjected to a surgery and/or chemotherapy and/or radiotherapy,
b. Determination of the level of the secreted, extra cellular form of the soluble part of the (pro)renin receptor as set forth by SEQ ID NO:1 or fragments thereof, as determined by an Elisa test, in a patient having a tumoral and/or proliferative disorders of the central nervous system, in particular glioblastomas before said surgery and/or chemotherapy and/or radiotherapy,
c. Comparison of both values, the value determined in step a. being lower than the one of step b. being indicative of a the efficacy of surgery and/or chemotherapy and/or radiotherapy.

Thus in this embodiment, said process is carried out *ex vivo.* Disclosed is an antibody specifically binding to one of the amino acid sequence set forth by SEQ ID 4-8.

In another aspect, the present invention relates to the use of a kit comprising an antibody specifically binding to one of the amino acid sequence set forth by SEQ ID 4-8 for the *in vitro* determination of the level of the secreted, extra cellular form of the soluble part of the (pro)renin receptor as set forth by SEQ ID NO:1 or fragments thereof, and means for the detection of the immune complex formed between said antibody and said secreted, extra cellular form of the soluble part of the (pro)renin receptor as set forth by SEQ ID NO:1 or fragment thereof.

In, an advantageous embodiment, said biological fluid used in the kit defined above is the cerebrospinal fluid.

### DESCRIPTION OF THE FIGURES

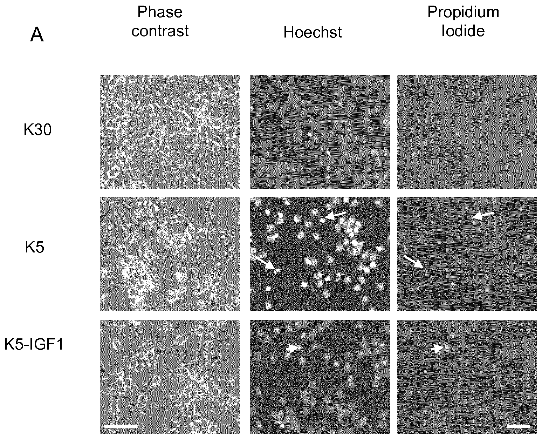

**Figures 1A to 1C** present the induction of apoptosis in isotope-labeled CGN cultures and its rescue by KCl depolarization or IGF-1 treatment. CGNs grown for 6 days in culture medium containing isotope-labeled arginine and lysine were switched for 12 h to serum-free BME containing labeled lysine and arginine and 5 mM KCl (K5), 30 mM KCl (K30) or 5 mM KCl + 100 ng/ml IGF-1 (K5-IGF1).
Figure 1A: Phase contrast and fluorescence photomicrographs of representative fields are shown. Dense and fragmented nuclei visualized by Hoechst 33258 staining and not labeled by propidium iodide (arrows) were considered as apoptotic. Propidium iodide-labeled nuclei corresponding to necrotic neurons are indicated by arrowheads. Scale bar: 100 µm. (From left to right column: Phase contrast, Hoechst, Propidium iodide; from the top to bottom line: K30, K5, K5-IGF-1)

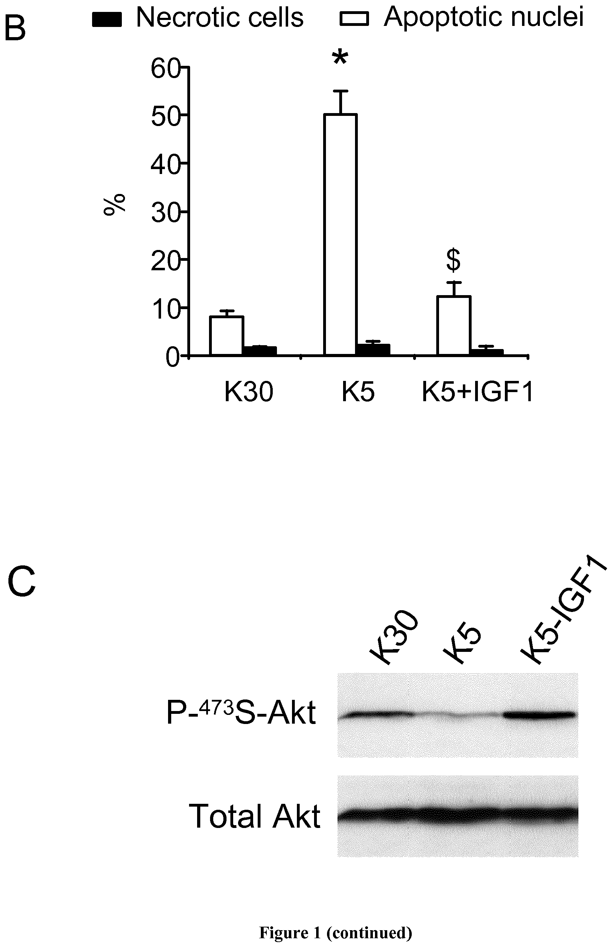

Figure 1B: Quantification of the percentage of apoptotic nuclei and necrotic cells in the three experimental conditions. Data are the mean ± SEM of values obtained in three independent cultures (three fields containing at least 200 nuclei counted in each culture). * p < 0.01 *vs*. K30, $ p < 0.01 *vs*. K5, ANOVA followed by Student-Newman-Keuls test (black histogram: necrotic cells, white histograms: apoptotic nuclei; x-axis: from left to right histograms: K30, K5, K5 + IGF-1; y-axis: % of apoptotic nuclei and necrotic cells).
Figure 1C: Phosphorylation of Akt (protein kinase B) at Ser473 was evaluated by immunoblotting. A Western blot representative of three independent experiments performed using different sets of cultured CGNs is illustrated (from left to right: K30, K5, K5 + IGF-1).

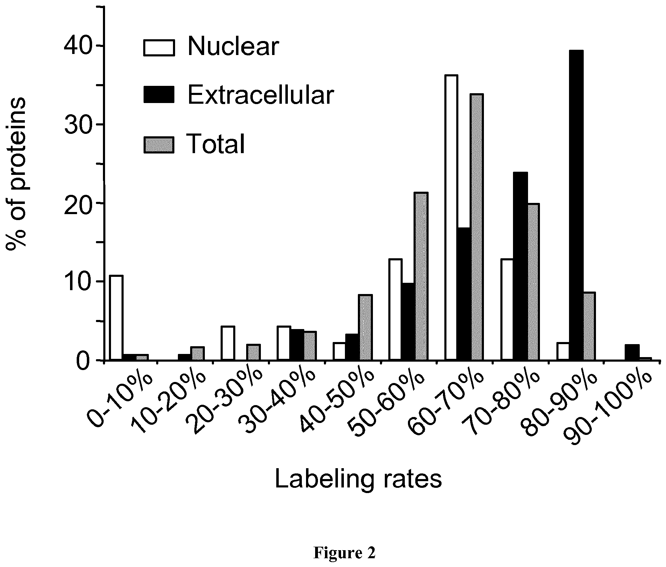

**Figure 2** **presents the variations in heavy isotope incorporation into proteins from CGN conditioned media**
   Supernatants of CGNs grown in the presence of either Arg6 and Lys4 or Arg10 and Lys8 were mixed (1:1 protein ratio) and samples were analyzed by LC-FT-MS/MS as indicated in "Examples". The histogram shows the isotope enrichment ratios for all the quantified proteins (Total) and for the proteins annotated as nuclear or extracellular. The *y*-axis represents the percentage of proteins of each category exhibiting the indicated isotope incorporation rate and the x-axis the labeling rate. (white histograms: Nuclear, black histograms: extracellular, grey histograms: total).
**Figures 3A to 3D** **present the differential expression of proteins in CGN-conditioned media upon apoptosis induction**
Figure 3A: The scatter plots of K30/KS (left panel) and K5-IGF1/K5 (right panel) protein ratios determined using two independent sets of cultures show a good reproducibility of the SILAC approach based on double labeling (Left part: x-axis log₂(K30/K5 ratio 1), y-axis: log₂(K30/K5 ratio 2); right part: x-axis log₂(K5-IFG-1/K5 ratio 1), y-axis: log₂(K5-IFG-1/K5 ratio 2).
Figure 3B: Intensity-ratio distribution of protein abundances in CGN-conditioned media. Protein ratios were plotted against the summed peptide intensities. Left panel: comparison between neurons switched to K30 or K5, right panel: comparison between neurons switched to K5-IGF1 or K5. The MaxQuant significance thresholds based on significance B (p <0.01) are depicted (Left part: x-axis log₂(intensity), y-axis: log₂(K30/K5 ratio 1); right part: x-axis log₂(intensity), y-axis: log₂(K5-IFG-1/K5 ratio 1).
Figure 3C: The scatter plot comparing the K30/K5 and K5-IGF1/K5 protein ratios shows a reasonable correlation between the changes in protein abundances in the two experimental conditions (x-axis log₂(K5-IFG-1/K5 ratio 1), y-axis: log₂(K30/K5 ratio 1).
Figure 3D: Venn diagram of the proteins that show significantly different K30/K5 and/or K5-IGF1/K5 SILAC ratios in both replicates. Note that 11 proteins were significantly regulated in the same way by K+-induced depolarization (K30) and IGF-1 treatment, in particular SPRR (SEQ ID NO: 1).
**Figures 4A and 4B** **present the validation by Western blotting of the modification of sPRR level upon induction of CGN apoptosis**
   CGNs grown in 35-mm culture dishes were switched to serum-free BME containing labeled lysine and arginine and 30 mM KCl (K30), 5 mM KCl (K5) or 5 mM KCl + 100 ng/ml IGF-1 (K5-IGF1) for 12 h and then conditioned media were harvested. Expression of (pro)renin receptor (Renin R) set forth by SEQ ID NO: 1 was analyzed by Western blotting.

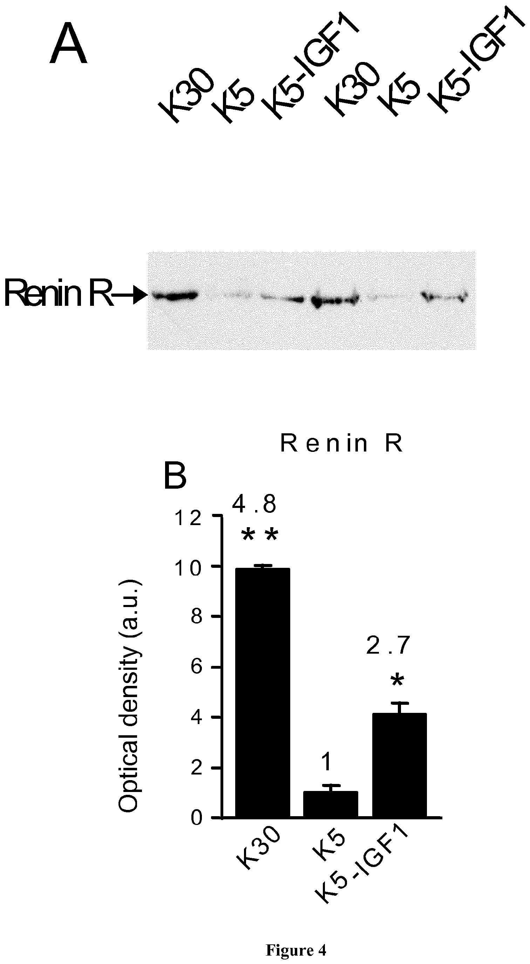

Figure 4A: Western blot representative of three independent experiments performed in duplicate using different sets of cultured CGNs (from left to right: K30, K5, K5-IGF-1, K30, K5, K5-IGF-1).
**Figure 4B****:** Quantification of the immunoreactive bands by densitometry using the Image J software. Data, normalized to the K5 values, are the mean ± SEM of the results obtained in three independent experiments. The numbers above the bars indicate the corresponding SILAC ratios. * p< 0.05, ** p < 0.01 *vs.* K5 (ANOVA followed by Dunnett's test) (x-axis: from left to right: K30, K5, K5-IGF-1, y-axis: optical density (a.u.).

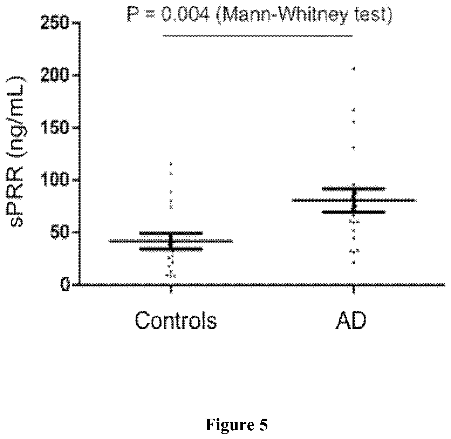

**Figure 5** presents the determination of sPRR levels in human CSF previously collected from patients afflicted by Alzheimer's disease and "control subjects" as determined by an Elisa test according to example 11.
   x-axis: From left to right: Controls (subjected to lumbar punctures following various neurological symptoms, e.g. headhache, migraine, subjective memory complaint but normal cognitive status and hydrocephalia); AD: patients with Alzheimer's disease.
   y-axis: level of sPRR in ng/mL.

### EXAMPLES:

### EXAMPLE 1: Primary cultures of mouse CGNs

Primary cultures of CGNs were prepared from 7 day-old mice (C57B1/6J, Janvier, Le Genest-St-Isle, France) using the procedure described by Desagher *et al.* (Desagher, S., Severac, D., Lipkin, A., Bernis, C., Ritchie, W., Le Digarcher, A., and Journot, L. (2005) Genes regulated in neurons undergoing transcription dependent apoptosis belong to signaling pathways rather than the apoptotic machinery. J Biol Chem 280, 5693-5702). Animals were handled following the guidelines of the French Ethics Committee (animal experimentation authorization number 34-71). Freshly dissected cerebella were incubated at 37°C with 0.25 mg/ml trypsin for 10 min and cells were mechanically dissociated in Hanks' balanced salt solution without Ca2+ and Mg2+ in the presence of 0.5 mg/ml trypsin inhibitor and 0.1 mg/ml DNase I by using a fire narrowed Pasteur pipette. The resulting cell suspension was centrifuged and resuspended in Eagle's basal medium (BME) without glutamine, lysine and arginine (Euromedex, Souffelweyersheim, France) supplemented with 10% fetal bovine serum, 2 mM L-glutamine, 10 mM HEPES, 100 IU/ml penicillin, 100 µg/ml streptomycin, 25 mM KCl (to obtain a final concentration of 30 mM) and with L- [13C6]arginine (Arg6) and L-[2H4]lysine (Lys4), or L-[13C6-15N4]arginine (Arg10) and L- [13C6-15N2]lysine (Lys8) (Euriso-top, Saint-Aubin, France). The cell suspension was filtered through a Falcon 40-µm cell strainer and seeded at a density of 25 x 104 cells/cm2 in culture dishes coated with poly-D-lysine (BD Biosciences, Le Pont de Claix, France). Cells were grown at 37 °C in a humidified atmosphere in presence of 6% CO2 and 94% air for 6 days. To prevent proliferation of non-neuronal cells, cytosine β-D-arabinofuranoside (Sigma, St Quentin Fallavier, France) was added to the culture medium to a final concentration of 10 µM 24 h after seeding. At day 6, granule neurons represented more than 98% of the cells in the cultures (Desagher, S., Severac, D., Lipkin, A., Bernis, C., Ritchie, W., Le Digarcher, A., and Journot, L. (2005) Genes regulated in neurons undergoing transcription dependent apoptosis belong to signaling pathways rather than the apoptotic machinery. J Biol Chem 280, 5693-5702).

### EXAMPLE 2: Induction of CGN apoptosis and media conditioning

Cells grown in 100-mm culture dishes were washed four times with 10 ml serum-free BME without lysine and arginine and supplemented with 1 mM L-glutamine, 5 mM HEPES buffer (pH 7.4), antibiotics, 1 µM of the *N*-methyl-D-aspartate receptor antagonist (+)-MK-801 (Sigma) and 30 mM KCl. (+)-MK-801 was added to the medium to avoid changes in protein expression due to uncontrolled *N*-methyl-D-aspartate receptor stimulation by endogenously released glutamate. Cells were then incubated with a minimal volume of the same medium (6 ml for cells in 100-mm dishes) in the presence of 30 mM KCl + Arg10 and Lys8 (K30), or 5 mM KCl and 100 ng/ml IGF-1 + Arg10 and Lys8 (K5-IGF1), or 5 mM KCl + Arg6 and Lys4 (K5) at 37°C and 5 % CO2 for 12 h. This incubation period allowed the accumulation in the medium of sufficient protein amounts for mass spectrometry analysis (∼ 3.5 µg protein/ml) with minimal apoptotic (in the K30 and K5-IGF1 conditions) and necrotic neuronal death (about 2-3% in all conditions) (see Fig. 1). After the 12-h secretion period, CGN-conditioned media were harvested and centrifuged at 200 g for 5 min and then at 20,000 g for 25 min to remove non-adherent cells and cell debris. Supernatants were kept at -80°C. Protein concentration in CGN supernatants was determined using the Micro BCA protein assay kit (Thermo Fisher Scientific).

### EXAMPLE 3: Measurement of CGN necrosis and apoptosis

Necrosis of CGNs was assessed by measuring nuclei staining with propidium iodide (PI). PI is a fluorescent intercalating agent that does not permeate intact plasma membranes and therefore does not label nuclei of living or apoptotic cells. After PI staining (3 µg/ml, 10 min, Sigma), CGNs were fixed with 4% paraformaldehyde in PBS (10 min at 4 °C) and nuclei were stained with 1 µg/ml Hoechst 33258 (Sigma) at room temperature for 10 min. Cells were then washed with PBS and distilled water and mounted in Mowiol under coverslips. Nuclear DNA staining was examined by fluorescence imaging microscopy using an Axiophot2 microscope (Carl Zeiss, Le Pecq, France) equipped with epifluorescence. Images were acquired with a CoolSNAP CCD camera (Photometrics) driven by the Metamorph software (Molecular Devices, Sunnyvale, CA) and transferred to Adobe Photoshop (version 7) for image processing. Necrosis was estimated by counting the number of PI-positive nuclei relative to the total number of nuclei (stained with Hoechst 33258) in at least nine different fields (about 350 cells per field) from three independent cultures. Condensed or fragmented nuclei visualized by Hoechst 33258 were considered as apoptotic.

### EXAMPLE 4: Gel and LC-MS/MS analysis of CGN conditioned media

Supernatants from five sets of CGNs grown independently in 100 mm culture dishes (corresponding to ∼ 50 µg proteins) were used for each experimental condition. Equal protein amounts of samples to be compared were mixed and concentrated by precipitation with 10% (wt/v) trichloroacetic acid (TCA) at 4°C for 2 h. TCA precipitates were solubilized in SDS sample buffer (62.5 mM Tris-HCl, pH 6.8, 2% SDS, 10% glycerol, 1% 2- mercaptoethanol and 0.005% bromophenol blue) and proteins were resolved on 12%-18% gradient gels using the Protean II xi Cell system (Bio-Rad Laboratories, Marnes la Coquette, France). Gels were stained with PageBlue Protein Staining Solution (Fermentas, Vilnius, Lithuania) and scanned using a computer-assisted densitometer (Epson Perfection V750 PRO). Gel lanes were systematically cut into 20 regular pieces and destained with three washes in 50% acetonitrile. After protein reduction (with 10 mM dithiothreitol at 56°C for 15 min) and alkylation (55 mM iodoacetamide at room temperature for 30 min), proteins were in-gel digested using trypsin (600 ng/band, Gold, Promega, Charbonnières, France), as previously described (Thouvenot, E., Urbach, S., Dantec, C., Poncet, J., Seveno, M., Demettre, E., Jouin, P., Touchon, J., Bockaert, J., and Marin, P. (2008) Enhanced detection of CNS cell secretome in plasma protein-depleted cerebrospinal fluid. J Proteome Res 7, 4409-4421). Digested products were dehydrated in a vacuum centrifuge and reduced to 2 µl. The generated peptides were analyzed online by nano-flow HPLCnanoelectrospray ionization using a LTQ Orbitrap XL mass spectrometer (Thermo Fisher Scientific, Bremen, Germany) coupled to an Ultimate 3000 HPLC (Dionex, Thermo Fisher Scientific). Desalting and pre-concentration of samples were performed on-line on a Pepmap® pre-column (0.3 mm x 10 mm, Dionex). A gradient consisting of 0-40% B in 60 min, 40-80% B in 15 min (A = 0.1% formic acid, 2% acetonitrile in water; B = 0.1 % formic acid in acetonitrile) at 300 nl/min was used to elute peptides from the capillary reverse-phase column (0.075 mm x 150 mm, Pepmap®, Dionex). Eluted peptides were electrosprayed online at a voltage of 2.4 kV into an LTQ Orbitrap XL mass spectrometer. A cycle of one full-scan mass spectrum (460-1,600 m/z) at a resolution of 60,000, followed by five data-dependent MS/MS spectra was repeated continuously throughout the nanoLC separation. All MS/MS spectra were recorded using normalized collision energy (35 %, activation Q: 0.25 and activation time 30 ms) and an isolation window of 2 m/z. Data were acquired using the Xcalibur software (v 2.0.7).

### EXAMPLE 5: Protein identification and quantification

Raw data were analyzed using the MaxQuant software (version 1.0.13.13) with standard settings (Cox, J., and Mann, M. (2008) MaxQuant enables high peptide identification rates, individualized p.p.b.-range mass accuracies and proteome-wide protein quantification. Nat Biotechnol 26, 1367-1372). The generated peak lists were searched against a concatenated mouse database by using the Mascot v2.2 algorithm (Matrix Science Inc., Boston, USA). The Uniprot mouse database (release 15.7; http://www.expasy.ch, 127,998 entries), to which frequently observed contaminants and reversed sequences of all entries were added (see (Cox, J., and Mann, M. (2008) for details), was used. All searches were carried out with precursor mass tolerance of 7 ppm, fragment mass tolerance of ± 0.5 Th, methionine oxidation as variable modification, SILAC modification and two trypsin missed cleavages allowed. Only peptides with at least six amino acids in length were considered.

Peptide identifications were accepted based on their posterior error probability (less than 1%). Accepted peptide sequences were subsequently assembled by MaxQuant into proteins to achieve a false discovery rate of 1% at the protein level. For protein identification, at least two peptides (among which at least one unique peptide) were required. Relative protein quantifications in each sample were performed based on the median SILAC ratios of at least two peptides, using MaxQuant with standard settings. Significance thresholds were also calculated by MaxQuant based on significance B with a *p* value of 0.01 for normalized peptide ratios (Cox, J., and Mann, M. (2008) MaxQuant enables high peptide identification rates, individualized p.p.b.-range mass accuracies and proteome-wide protein quantification. Nat Biotechhol 26, 1367-1372). Graphical representations were generated using the R statistical environment (V2.9.1) (Team, R. D. C. (2009) R: A language and environment for statistical computing., R Foundation for Statistical Computing, Vienna, Austria).

### EXAMPLE 6: Western blot analysis

Proteins from CGN supernatants were concentrated by precipitation with 10% TCA. Proteins (30 µg per lane) were resolved on 11-17% gradient gels and transferred electyrophoretically to Hybond C nitrocellulose membranes (GE Healthcare). Membranes were immunoblotted with primary antibodies (rabbit anti-(pro)renin receptor 1:500 (Cousin, C., Bracquart, D., Contrepas, A., Corvol, P., Muller, L., and Nguyen, G. (2009) Soluble form of the (pro)renin receptor generated by intracellular cleavage by furin is secreted in plasma. Hypertension 53, 1077-1082) or antibodies directed toward Ac45 (rabbit IgG 1623 et 1645, internal names) and then with either anti-mouse or anti-rabbit horseradish peroxidase-conjugated secondary antibodies (1:3,000, Amersham Biosciences, Saclay, France). Immunoreactivity was detected with an enhanced chemiluminescence method (ECLTM plus detection reagent, Amersham Biosciences) and immunoreactive bands were quantified by densitometry using the ImageJ software (v1.44). For measurement of Akt activation, CGNs grown in 35-mm culture dishes were exposed to either K30, K5 or K5-IGF1 for 12 h. Cells were then lysed in 50 mM Tris- HCl, pH 7.4, 1 mM EDTA and 1% SDS. Protein concentration was determined using the Micro BCA protein assay kit. Proteins were resolved on 12% polyacrylamide gels and transferred to nitrocellulose membranes. Akt activation was analyzed by sequential immunoblotting with an antibody that recognizes specifically Akt phosphorylated at Ser473 (1:1,000 dilution, Cell Signaling Technology, Ozyme, Saint Quentin en Yvelines) and an antibody against total Akt (1:1,000 dilution, Cell Signaling Technology).

### Example 7: Induction of apoptosis in isotope-labeled CGN cultures

As CGNs are non-dividing cells that can only be cultured in the presence of non-dialyzed serum, optimal isotopic labeling of proteins (> 95%) cannot be achieved in these cultures. To overcome bias in protein quantification due to incomplete labeling and to get a global picture of the changes in protein secretion by neurons during apoptosis, CGN cultures were grown in presence of either Arg10 and Lys8 (K30 and K5-IGF1 condition) or Arg6 and Lys4 (K5 condition) amino acids for the entire experimental period (6-day culture and 12-h apoptosis induction). As previously shown for cultures grown in the presence of unlabeled amino acids (Desagher, S., Severac, D., Lipkin, A., Bernis, C., Ritchie, W., Le Digarcher, A., and Journot, L. (2005) Genes regulated in neurons undergoing transcription-dependent apoptosis belong to signaling pathways rather than the apoptotic machinery. J Biol Chem 280, 5693-5702, Lafon-Cazal, M., Perez, V., Bockaert, J., and Marin, P. (2002) Akt mediates the anti-apoptotic effect of NMDA but not that induced by potassium depolarization in cultured cerebellar granule cells. Eur J Neurosci 16, 575-583), switching isotope-labeled CGNs to K5 in absence of serum for 12 h increased the number of apoptotic neurons, when compared to cultures switched to K30 (48 ± 4.7 % vs. (48 ± 1.4 % of apoptotic nuclei, respectively, n = 3, Fig. 1*A*, *B*). Addition of 100 ng/ml IGF-1 in K5 (K5-IGF1) prevented CGN apoptosis to the same extent as in cultures switched to K30 (48 ± 2.8 % of apoptotic nuclei, n = 3). Consistent with previous findings (Lafon-Cazal, M., Perez, V., Bockaert, J., and Marin, P. (2002) Akt mediates the anti-apoptotic effect of NMDA but not that induced by potassium depolarization in cultured cerebellar granule cells. Eur J Neurosci 16, 575-583), these treatments did not induce necrotic cell death, as indicated by the lack of PI incorporation in unfixed neurons (Fig. 1*A*, *B*). Correspondingly, phosphorylation of Akt (at Ser473) was more elevated in CGNs switched to K30 or K5-IGF1 than in CGNs exposed to K5 (Fig. 1*C*), indicating that both K+-induced depolarization and IGF-1 exposure engage the anti-apoptotic Akt signaling pathway in isotope-labeled CGNs, as previously shown in unlabeled neurons (Lafon-Cazal, M., Perez, V., Bockaert, J., and Marin, P. (2002) Akt mediates the anti-apoptotic effect of NMDA but not that induced by potassium depolarization in cultured cerebellar granule cells. Eur J Neurosci 16, 575-583).

### Example 8: Quantitative analysis of variations in protein secretion induced by apoptosis

After the 12-h period of apoptosis induction, CGN-conditioned media were harvested and 1:1 mixtures of differentially labeled samples were subjected to a SDS-PAGE and in gel digestion with trypsin. Peptides were analyzed by LC-FT-MS/MS and the relative quantification of precursor ion signals was performed using the MaxQuant software. A median labeling rate of 62% for all quantified proteins was found. However, large differences in isotope incorporation rates were measured from one protein to one another (Fig. 2). Specifically, proteins with a known extracellular location, which include proteins secreted *via* the vesicular pathway as soon as they are synthesized, showed the highest median labeling rate (77%). In contrast, proteins that are mainly localized in the nucleus and that were recovered in the cell supernatant as a result of their release by the minority of necrotic neurons present in the cultures, showed the lowest median labeling rate (60%), which probably reflected their low turnover in non-dividing neurons. These variations in the incorporation of heavy isotopes during the culture and apoptosis induction periods strongly support the choice of using a double labeling procedure for accurate quantification of individual proteins released by neurons.

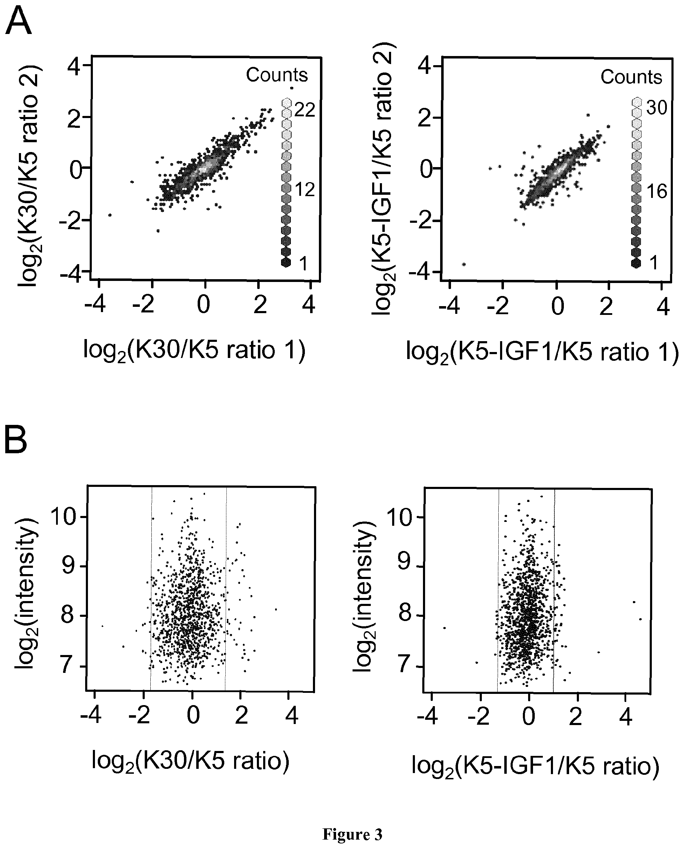

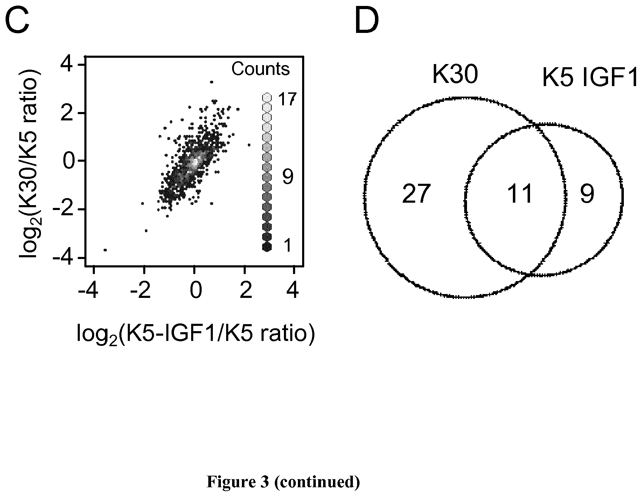

A total of 1,842 proteins were quantified (at least in one replicate) in conditioned media from cells cultured in K5 (apoptosis condition) and in K30 or K5-IGF1 (cell survival conditions). The correlation plots in which the K30/K5 and K5-IGF1/K5 protein abundance ratios (SILAC ratios) were compared in biological replicates showed that protein quantification was highly reproducible (Fig. 3*A*). Overall, 1,005 proteins were quantified in all four independent experiments (K30 vs. K5 and K5-IGF1 *vs*. K5 in duplicate) performed in the present study. Most of the quantified proteins did not exhibit significant changes in expression level upon apoptosis induction, based on significance B with a *p* value of 0.01 (Fig. 3*B*). Only 47 proteins showed significantly different K30/K5 and/or K5-IGF1/K5 SILAC ratios in both duplicates and sPRR is presented in Table I and the abundance of it was higher in the K30 or the K5-IGF1 supernatants than in the K5 samples.

Moreover, comparison of the K30/K5 and K5-IGF1/K5 abundance ratios revealed that the changes in protein secretion induced by K30 and IGF1 were correlated (Fig. 3*C*).

Accordingly, exposure to K30 or IGF1 induced the same type of quantitative variation in secretion (i.e., increase or decrease) for the two differentially expressed proteins of Table I and eleven proteins showed significant SILAC ratios in both conditions (K30/K5 and K5-IGF1/K5, Fig. 3D).

**Table I: Proteins exhibiting significant K30/K5 and/or K5-IGF1/K5 SILAC ratios in both biological replicates**

| Protein ID | Protein Name | MW (kDa) | Peptide Count | Cover (%) | K30/ K5 ratio 1 | K30/ K5 ratio 2 | K5-IGF1/ K5 ratio 1 | K5-IGF1/ K5 ratio 2 | Human CSF ID | Cellular location or NN-score |
|---|---|---|---|---|---|---|---|---|---|---|
| Q9CY N9 | Renin receptor (SEQ ID NO:1) | 39.1 | 9 | 32 | 4.79* | 4.83* | 2.79* | 2.55* | 07578 7 | Membrane |

Protein IDs correspond to the leading protein. Protein names are those from the UniProt database. Peptide count and cover indicate the number of peptides quantified by MaxQuant for each protein and the corresponding sequence coverage. K30/K5 ratio 1 and 2 and K5-IGF1/K5 ratio1 and 2 represent the protein ratios measured in individual experiments.* Indicates significant ratios (p < 0.01),
For proteins also identified in CSF, the human ID is indicated, 'Secreted' and 'Membrane' indicate proteins annotated as secreted or transmembrane/membrane associated proteins in the UniProt database, respectively. The NNscore corresponds to the score provided by the SecretomeP 2.0 web server (http://www.cbs.dtu.dk/services/SecretomeP/). A score higher than 0.5 indicates a high probability of secretion *via* a non-classical secretion mechanism.

### Example 9: Validation of differentially secreted proteins by Western blotting

In order to validate the quantitative proteomic data, the expression of proteins with significant SILAC ratios was then analyzed by Western blotting. The soluble form of (pro)rennin receptor, a recently described receptor specific for renin and prorenin that exerts angiotensin II-dependent and independent functions (Nguyen, G., Delarue, F., Burckle, C., Bouzhir, L., Giller, T., and Sraer, J. D. (2002) Pivotal role of the renin/prorenin receptor in angiotensin II production and cellular responses to renin. J Clin Invest 109, 1417-1427) and Ac45 were focused.

The choice was based on 1) the availability of well-characterized antibodies for Western blotting 2) their upregulation in CGN supernatants in both survival conditions and 3) their potential neuroprotective effect in neurodegenerative diseases (Mattson, M. P., Cheng, B., Culwell, A. R., Esch, F. S., Lieberburg, I., and Rydel, R. E. (1993) Evidence for excitoprotective and intraneuronal calciumregulating roles for secreted forms of the beta-amyloid precursor protein. Neuron 10, 243-254 ; Gauthier, S., Kaur, G., Mi, W., Tizon, B., and Levy, E. (2011) Protective mechanisms by cystatin C in neurodegenerative diseases. Front Biosci (Schol Ed) 3, 541-554 ; Valenzuela, R., Barroso-Chinea, P., Villar-Cheda, B., Joglar, B., Munoz, A., Lanciego, J. L., and Labandeira-Garcia, J. L. (2010) Location of prorenin receptors in primate substantia nigra: effects on dopaminergic cell death. J Neuropathol Exp Neurol 69, 1130-1142).

Western blot analysis confirmed that (pro)renin receptor was significantly upregulated in the supernatants of CGNs switched to K30 or K5-IGF1 in comparison to conditioned medium from cells exposed to K5 (Fig. 4).

### EXAMPLE 10: preparation of the monoclonal antibodies

### Supplier Eurogentec for antibodies directed toward sequence set forth by SEQ ID NO: 4-8

### Monoclonal antibody

Phase I - Immunisation - per mouse (minimum 4 mice)
Phase II - Fusion - Eight 96 well plates
Phase III - Analysis - 8 plate fusion
Phase IV - Cloning and isotyping - 1 or 2 hybridoma

### Phase I: Immunization

- A minimum of four mice (6-8 weeks old) is used for immunization. Preimmune serum is taken on day 0.
- A total of four injections are administered (50 µg antigen per animal per injection). The first injection on day 0 with equal parts (v/v) of complete Freund's adjuvant and antigen. The following three boosts are carried out with equal parts (v/v) incomplete Freund's adjuvant and antigen on days 21 and 42 with an additional final boost prior to fusion.
- Selection of the best responding mouse 10 days after the last injection using the client's ELISA test.

If the antibody titer is too low after three booster injections, you may request additional injections. If the ELISA report remains negative, the immunization should be terminated because the probability of success is too low.

If the antigen has a low immunogenicity, it can be coupled to an appropriate protein carrier.

Duration of this standard program: 11 weeks. The above description is the Eurogentec standard protocol. However, a personalized procedure may be designed and adapted to your needs.

### Phase II: Fusion

The splenic lymphocytes of the best responding animal will be fused with the cell line Sp2/O-Ag-14 using PEG (polyethylene glycol), and the resulting hybridomas will be seeded in eight 96-well plates in HAT medium. If any fusion cocktail remains, it will be stored in liquid nitrogen.

### Duration: 2-3 weeks

### Phase III: Screening and amplification of hybridomas

- IgG producing hybridomas will be screened against the target antigen and two vials of every positive hybridoma stored in liquid nitrogen
- The positive colonies are expanded and tested again for antibody production with the antigen used for immunization
- At this point, positive colonies can also be screened against additional antigen for cross-reactivity or against a region of the antigen of interest
- Other screening tests can be used such as Western blots or immunofluorescence. Please contact us for a price offer
- In addition, 5-10 ml supernatant of the 20 most positive hybridomas will be sent to you for screening confirmation and additional tests. This will ensure that the positive ELISA results identify antibodies useful for your purpose

### Duration: 5-7 weeks

### Phase IV: Cloning and isotyping

- The specific hybridomas you have selected at the end of Phase III will be cloned by limit dilution. The assay can be carried out as in Phase III (screening for the antigen used for immunization and additional screening of positive clones with additional antigens). After cloning, three vials of every cloned hybridoma are stored in liquid nitrogen. We send you 15-20 ml of every hybridoma supernatant together with the frozen hybridomas.
- Subsequently, the subclass of every monoclonal antibody will be determined by ELISA testing.

### Duration: 4 weeks

### EXAMPLE 11: Dosage of sPRR (SEQ ID NO:1) in human CSF (control patients and patients afflicted by an Alzheimer's disease).

sPRR has been measured with an Elisa test according to the manufacturer's protocol depending on the coupled antibodies (directed toward sequence set forth by SEQ ID NO: 4-8) but the starting dilution of CSF is 1/25.

### Human soluble (Pro)renin Receptor Assay Kit - IBL

### PRINCIPLE

This kit is a solid phase sandwich ELISA using 2 kinds of highly specific antibodies.

Tetra Methyl Benzidine (TMB) is used as a coloring agent (Chromogen). The intensity of coloring is proportional to the quantities of Human (Pro)renin Receptor.

### KIT COMPONENT

1- Precoated plate :
   Anti-Human (Pro)renin receptor Rabbit IgG Affinity Purify 96Well x 1
2- Labeled antibody Conc.:
   (30X) HRP conjugated Anti- Human (Pro)renin receptor (93A1B) Mouse IgG Fab' Affinity Purify 0.4mL x 1
3- Standard : Recombinant human (Pro)renin Receptor 0.5mL x 2
4- EIA buffer : 1% BSA, 0.05% Tween20 in PBS 30mL x 1
5- Solution for Labeled antibody: 1% BSA, 0.05% Tween20 in PBS 12mL x 1
6- Chromogen : TMB solution 15mL x 1
7- Stop solution : 1N H2SO4 12mL x 1
8- Wash buffer Conc. : (40X) 0.05% Tween20 in phosphate buffer 50mL x 1

### OPERATION MANUAL

### 1. Materials needed but not supplied

- Plate reader (450nm) · Micropipette and tip
- Graduated cylinder and beaker · Deionized water
- Refrigerator (as 4°C) · Graph paper (log/log)
- Paper towel · Tube for dilution of Standard
- Washing bottle for precoated plate
- Disposable test tube for "2, Labeled antibody Conc." and "6, Chromogen"

### 2. Preparation

### 1) Preparation of wash buffer

"8, Wash buffer Conc." is a concentrated (40X) buffer. Adjust the temperature of "8, Washing buffer Conc." to room temperature and then, mix it gently and completely before use. Dilute 50 mL of "8, Wash buffer Conc." with 1,950 mL of deionized water and mix it. This is the wash buffer for use.

This prepared wash buffer shall be stored in refrigerator and used within 2 weeks after dilution.

### 2) Preparation of Labeled antibody

"2, Labeled antibody Conc." is a concentrated (30X). Dilute "2, Labeled antibody Conc." with "5, Solution for Labeled antibody" in 30 times according to required quantity into a disposable test tube. Use this resulting solution as Labeled antibody.

### Example)

In case you use one strip (8 well), the required quantity of Labeled antibody is 800 µL. (Dilute 30 µL of "2, Labeled antibody Conc." with 870 µL of "5, Solution for Labeled antibody" and mix it.

And use the resulting solution by 100 µL in each well.)

This operation should be done just before the applying of Labeled antibody.

The remaining "2, Labeled antibody Conc." should be stored at 4°C in firmly sealed vial.

### 3) Preparation of Standard

Put just 0.5 mL of deionized water into the vial of "3, Standard" and mix it gently and completely. This solution is 16,000 pg/mL Human (Pro)renin Receptor standard.

### 4) Dilution of Standard

Prepare 8 tubes for dilution of "3, Standard". Put 230 µL each of "4, EIA buffer" into the tube.

Specify the following concentration of each tube."

Put 230 µL of Standard solution into tube-1 and mix it gently. Then, put 230 µL of tube-1 mixture into tube-2. Dilute two times standard solution in series to set up 7 points of diluted standard between 8,000 pg/mL and 125 pg/mL. Tube-8 is the test sample blank as 0 pg/mL.

### 5) Dilution of test sample

Test samples should be diluted with "4, EIA buffer" as necessary.

If the concentration of human (Pro)renin Receptor in samples may not be estimated in advance, the pre-assay with several different dilutions will be recommended to determine the proper dilution of samples.

### 3. Measurement procedure

All reagents shall be brought to room temperature approximately 30 minutes before use. Then mix it gently and completely before use. Make sure of no change in quality of the reagents. Standard curve shall be prepared simultaneously with the measurement of test samples.

| **Reagents** | **Test Sample** | **Standard** | **Test Sample Blank** | **Reagent Blank** |
|---|---|---|---|---|
| | **Test sample 100 µL** | **Diluted standard (Tube 1-7) 100 µL** | **EIA buffer (Tube-8) 100 µL** | **EIA buffer 100 µL** |
| Incubation overnight at 4°C with plate lid | | | | |
| Washing 4 times | | | | |
| **Labeled antibody** | 100 µL | 100 µL | 100 µL | - |
| Incubation for 60 minutes at 4°C with plate lid | | | | |
| Washing 5 times | | | | |
| **Chromogen** | 100 µL | 100 µL | 100 µL | 100 µL |
| Incubation for 30 minutes at room temperature (shielded) | | | | |
| **Stop solution** | 100 µL | 100 µL | 100 µL | 100 µL |
| Read the plate at 450nm against a Reagent Blank within 30 minutes after addition of Stop solution. | | | | |

1) Determine wells for reagent blank. Put 100 µL each of "4, EIA buffer" into the wells.
2) Determine wells for test sample blank, test sample and diluted standard.
   Then, put 100 µL each of test sample blank (tube-8), test sample and dilutions of standard (tube-1-7)into the appropriate wells.
3) Incubate the precoated plate overnight at 4°C after covering it with plate lid.
4) Wash each well of the precoated plate 4 times with wash buffer using a washing bottle or a plate washer in following way.
   After shaking off (or aspiration of) the solution in wells, fill each well with wash buffer and shake off the wash buffer completely from the precoated plate.
   This procedure must be repeated 4 times. Then, drain the precoated plate completely on paper towel.
   Please refer to 5) and 6) in SPECIAL ATTENION below, and be careful not to miss a well.
5) Pipette 100 µL of labeled antibody solution into the wells of test samples, diluted standard and test sample blank.
6) Incubate the precoated plate for 60 minutes at 4°C after covering it with plate lid.
7) Wash the precoated plate 5 times in the same manner as 4).
8) Take the required quantity of "6, Chromogen" into a disposable test tube. Then, pipette 100 µL from the test tube into every well. Please do not return the rest of used chromogen in the test tube into "6, Chromogen" bottle in order to avoid contamination.
9) Incubate the precoated plate for 30 minutes at room temperature in the dark. The solution of Chromogen will turn blue.
10) Add 100 µL of "7, Stop solution" to all wells. Mix the solution by tapping the side of precoated plate. The solution will turn yellow by addition of "7, Stop solution".
11) Remove any dirt or drop of water on the bottom of the precoated plate and confirm there is no bubble on the surface of the solution. Then, run the plate reader and conduct measurement at 450 nm against a reagent blank.

The measurement shall be done within 30 minutes after addition of "7, Stop solution".

### SPECIAL ATTENTION

1) Test samples should be measured soon after collection. For the storage of test samples, store them frozen and do not repeat freeze/thaw cycles. Thaw the test samples at a low temperature and mix them completely before measurement.
2) Test samples should be diluted with "4, EIA buffer", as the need arises.
3) Duplicate measurement of test samples and standard is recommended.
4) Use test samples in neutral pH range. The contaminations of organic solvent may affect the measurement.
5) Use only wash buffer contained in this kit for washing the precoated plate.
   Insufficient washing may lead to the failure in measurement.
6) Remove the wash buffer completely by tapping the precoated plate on paper towel. Do not wipe wells with paper towel.
7) "6, Chromogen" should be stored in the dark due to its sensitivity against light.
   Avoid contact of Chromogen with metals.
8) Measurement should be done within 30 minutes after addition of "7, Stop solution".

### CALCULATION OF TEST RESULT

Subtract the absorbance of test sample blank from all data, including standards and unknown samples before plotting. Plot the subtracted absorbance of the standards against the standard concentration on log-log graph paper. Draw the best smooth curve through these points to construct the standard curve. Read the concentration for unknown samples from the standard curve.

The results on AD patients and controls are presented in table II below:

**TABLE II**

| **Sample code** | **Pathology** | **sPRR (ng/mL)** | **Group** | **Age** |
|---|---|---|---|---|
| DEM31LCR.2 | ALZHEIMER | 21,5 | Alzheimer | 39 |
| DEM61LCR.3 | ALZHEIMER | 75,24 | Alzheimer | 61 |
| DEM68LCR.1 | ALZHEIMER | 58,84 | Alzheimer | 75 |
| DEM207LCR.2 | ALZHEIMER | 66,08 | Alzheimer | 75 |
| DEM232LCR.3 | ALZHEIMER | 84,1 | Alzheimer | 53 |
| DEM234LCR.2 | ALZHEIMER | 32,39 | Alzheimer | 70 |
| DEM250LCR.2 | ALZHEIMER | 206,17 | Alzheimer | 66 |
| DEM255LCR.2 | ALZHEIMER | 95,57 | Alzheimer | 72 |
| DEM293LCR.3 | ALZHEIMER | 156,03 | Alzheimer | 66 |
| DEM302LCR.2 | ALZHEIMER | 31,27 | Alzheimer | 71 |
| DEM306LCR.1 | ALZHEIMER | 59,21 | Alzheimer | 38 |
| DEM350LCR.1 | ALZHEIMER | 72,93 | Alzheimer | 61 |
| DEM401 LCR.1 | ALZHEIMER | 60,88 | Alzheimer | 67 |
| DEM464LCR.1 | ALZHEIMER | 166,7 | Alzheimer | 65 |
| DEM521 LCR.1 | ALZHEIMER | 77,1 | Alzheimer | 56 |
| DEM599LCR.1 | ALZHEIMER | 87,49 | Alzheimer | 63 |
| DEM680LCR.1 | ALZHEIMER | 51,77 | Alzheimer | 62 |
| DEM844LCR.1 | ALZHEIMER | 33,1 | Alzheimer | 66 |
| DEM226LCR.1 | ALZHEIMER | 131,21 | Alzheimer | 69 |
| DEM635LCR.1 | ALZHEIMER | 44,48 | Alzheimer | 66 |
| **Mean** | | **80,60** | | **63** |
| | | | | |
| **SD** | | **11,29** | | |
| | | | | |
| SEP6LCR.2 | HEADACHE | 32,14 | Control | 46 |
| SEP765LCR.2 | DISC HERNIA | 8,69 | Control | 39 |
| SEP243LCR.2 | HYDROCEPHALIA | 43,73 | Control | 79 |
| SEP471 LCR.1 | HYDROCEPHALIA Intracranial hypertension | 40,78 | Control | 81 |
| DEM5LCR.1 | syndrome | 74,64 | Control | 43 |
| SEP377LCR.1 | HYDROCEPHALIA | 38,92 | Control | 58 |
| SEP1401 LCR.1 | MIGRAINES | 25,83 | Control | 37 |
| SEP454LCR.1 | MIGRAINES PSYCHIATRIC | 12,5 | Control | 48 |
| DEM81LCR.1 | SYNDROME | 17,98 | Control | 73 |
| DEM367LCR.1 | MEMORY COMPLAINT | 79,95 | Control | 58 |
| DEM430LCR.1 | MEMORY COMPLAINT | 106,05 | Control | 55 |
| DEM531 LCR.1 | MEMORY COMPLAINT | 88,5 | Control | 83 |
| DEM641 LCR.1 | MEMORY COMPLAINT | 26,28 | Control | 75 |
| DEM750LCR.1 | MEMORY COMPLAINT | 8,66 | Control | 66 |
| DEM757LCR.1 | MEMORY COMPLAINT | 21,04 | Control | 74 |
| DEM428LCR.1 | MEMORY COMPLAINT | 9,08 | Control | 70 |
| DEM65LCR.1 | SOMATIZATION | 27,58 | Control | 52 |
| SEP770LCR.2 | SPHINCTER DISORDER | 33,52 | Control | 43 |
| SEP832LCR.2 | SPHINCTER DISORDER | 22,12 | Control | 37 |
| SEP767LCR.2 | DIZZINESS | 115,76 | Control | 56 |
| **Mean** | | **41,69** | | **59** |
| | | | | |
| **SD** | | **7,57** | | |

Statistical test are presented on tables III and IV

### Statistical and normality test:

**TABLE III**

| **Groups** | **Control (ng/mL)** | **AD (ng/mL)** |
|---|---|---|
| Number of values | 20 | 20 |
| Minimum | 8.655 | 21.5 |
| 25% Percentile | 18.74 | 46.3 |
| Median | 29.86 | 69.5 |
| 75% Percentile | 66.91 | 93.55 |
| Maximum | 115.8 | 206.2 |
| | | |
| Mean | 41.69 | 80.60 |
| Std. Deviation | 33 | 49.18 |
| Std. Error | 7.38 | 11 |
| | | |
| Lower 95% Cl of mean | 26.24 | 57.64 |
| Upper 95% Cl of mean | 57.13 | 103.7 |
| | | |
| **D'Agostino& Pearson omnibus normality test** | | |
| K2 | 4.74 | 6.757 |
| P value | 0.0935 | 0.0341 |
| Passed normality test (alpha=0.05)? | **Yes** | **No** |
| P value summary | ns | * |

### Mean comparaison (AD v/s control):

**TABLE IV**

| | | |
|---|---|---|
| Table Analyzed | Data 1 | |
| Column A | Control | |
| vs | vs | |
| Column B | AD (ng/ml) | |
| | | |
| **Mann Whitney test** | | |
| **P value** | | **0.004** |
| Exact or approximate P value? | Gaussian Approximation | |
| P value summary | ** | |
| Are medians signif. different? (P < 0.05) | Yes | |
| One- or two-tailed P value? | Two-tailed | |
| Sum of ranks in column A,B | 303 , 517 | |
| Mann-Whitney U | | 93 |

In the CSF of control patients, a value of (48 ± 7.6 ng/mL was measured on samples from 20 control patients (mean age of 59) and 80.6 ± 11.3 ng/mL in 20 patients diagnosed as having Alzheimer's disease.

### Example 12: Dosage of sPRR (SEQ ID NO:1) in human plasma (control patients and patients afflicted by an Alzheimer's disease of example 11).

sPRR is determined in plasma of the patients by using the sPRR Elisa kit JP 27 782 commercialized by IBL (Hamburg) as described in example 11.

### Example 13: Dosage of sPRR (SEQ ID NO:1) in human CSF and plasma (control patients and patients afflicted by an Alzheimer's disease).

sPRR is determined as in example 11 and 12, in both CSF and plasma of a group, of about 300 patients diagnosed as having AD and 300 age matched controls suffering of various neurological disorders, as described in example 11.

## Claims

1. *In vitro* use of the secreted, extra cellular form of the soluble part of (pro)renin receptor as set forth by SEQ ID NO:1 or fragments thereof which bind to the renin or pro-renin, the level of which has been determined in a sample of a biological fluid previously collected in a human patient or an animal, as a pre-symptomatic dual biomarker of neurodegeneration and neuroregeneration, said biological fluid is the cerebrospinal fluid or the extracellular medium of any neuronal cell in primary culture, in particular in cerebellar granule neurons in primary culture, said level being modulated with respect to a sample of said biological fluid previously collected in a control human patient or a control animal.

2. Use of the level of the secreted, extra cellular form of the soluble part of the (pro)renin receptor as set forth by SEQ ID NO:1 or fragments thereof which bind to the renin or pro-renin, which has been determined *in vitro* in a sample of a biological fluid previously collected in a human patient or an animal, for the diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis, and/or
for the follow up of a treatment against a neurodegenerative disease in a patient afflicted by a neurodegenerative disease, in particular Alzheimer's disease, dementia with Lewy bodies, fronto-temporal dementia, Parkinson's disease, amyotrophic lateral sclerosis, and/or for the follow up of a treatment stimulating the neuroregeneration, and/or
for the screening of new drugs liable to treat neurodegenerative pathologies, in particular Alzheimer's disease, dementia with Lewy bodies, fronto-temporal dementia, Parkinson's disease, amyotrophic lateral sclerosis, and/or
for the diagnosis and/or the prognosis of tumoral and/or proliferative disorders of the central nervous system, in particular glioblastomas, and /or
for the follow-up of a treatment against tumoral and/or proliferative disorders of the central nervous system, in particular glioblastomas, after surgery and/or chemotherapy and/or radiotherapy
said level being modulated with respect to a sample of said biological fluid previously collected in a control patient or a control animal.

3. Use according to claim 2, in the diagnosis and/or the prognostic of a neurodegenerative disease or of a neurodegenerative disease stage, in particular Alzheimer's disease, dementia with Lewy bodies, fronto-temporal dementia, Parkinson's disease, amyotrophic lateral sclerosis,
wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than the one determined in control patients or animals, higher than 42 ng/mL, in particular higher than 50 ng/mL, as determined in control patients.

4. Use according to claim 2,
in the follow up of a treatment against a neurodegenerative disease in a human patient or animal, in particular Alzheimer's disease, dementia with Lewy bodies, fronto-temporal dementia, Parkinson's disease, amyotrophic lateral sclerosis,
wherein said fluid is the cerebrospinal fluid, in particular the human cerebrospinal fluid, in which the level of the secreted, extra cellular form of the soluble part of the (pro)renin receptor as set forth by SEQ ID NO: 1, as determined by an Elisa test, in a patient or animal previously treated with said treatment, is lower than the one determined in said patient or animal before said treatment,
or in the follow up of the treatment stimulating the neuroregeneration in a human patient or animal, wherein said fluid is the cerebrospinal fluid, in particular the human cerebrospinal fluid, in which the level of the secreted extra cellular form of the soluble part of the (pro)renin receptor as set forth by SEQ ID NO:1, as determined by an Elisa test, in a patient or animal previously treated with said treatment, is lower than 50 ng/mL.

5. Use according to claim 2, in the screening of new drugs liable to treat neurodegenerative pathologies in a human patient or animal, in particular Alzheimer's disease, dementia with Lewy bodies, fronto-temporal dementia, Parkinson's disease, amyotrophic lateral sclerosis,
wherein said fluid is the cerebrospinal fluid, in particular the human cerebrospinal fluid, in which the level of the secreted, extra cellular form of the soluble part of the (pro)renin receptor as set forth by SEQ ID NO: 1, as determined by an Elisa test, in a patient or animal afflicted by a neurodegenerative disease, previously treated with said new drug, is lower than the one determined in said patient or animal before the treatment with said new drug, or equal to the one determined in control patients.

6. Use according to claim 2, in the diagnosis and/or the prognosis of tumoral and/or proliferative disorders of the central nervous system, in particular glioblastomas,
wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, is higher than 42 ng/mL, in particular higher than 50 ng/mL, as determined in control patients or animals.

7. Use according to claim 2, in the follow-up of tumors of the central nervous system after surgery and/or chemotherapy and/or radiotherapy,
wherein said fluid is the cerebrospinal fluid, in particular the human cerebrospinal fluid, in which the level of the secreted, extra cellular form of the soluble part of the (pro)renin receptor as set forth by SEQ ID NO:1, as determined by an Elisa test, in a patient subjected to a surgery and/or chemotherapy is higher than the one determined in said patient or animal before surgery and/or chemotherapy, or equal to the one determined in control patients.

8. *In vitro* process for the *in vitro* diagnosis and/or the prognostic of a neurodegenerative disease or of a neurodegenerative disease stage in a patient liable to be afflicted by a neurodegenerative disease, in particular Alzheimer's disease, dementia with Lewy bodies, fronto-temporal dementia, Parkinson's disease, amyotrophic lateral sclerosis, and/or
for the follow up of a treatment against a neurodegenerative disease in a patient afflicted by a neurodegenerative disease, in particular Alzheimer's disease, dementia with Lewy bodies, fronto-temporal dementia, Parkinson's disease, amyotrophic lateral sclerosis, and/or for the follow up of a treatment stimulating the neuroregeneration, and/or
for the screening of new drugs liable to treat neurodegenerative pathologies, in particular Alzheimer's disease, dementia with Lewy bodies, fronto-temporal dementia, Parkinson's disease, amyotrophic lateral sclerosis, and/or
for the diagnosis and/or the prognosis of tumoral and/or proliferative disorders of the central nervous system, in particular glioblastomas, and /or
for the follow-up of a treatment against tumoral and/or proliferative disorders of the central nervous system, in particular glioblastomas, after surgery and/or chemotherapy and/or radiotherapy,
comprising the *in vitro* determination of the level of the secreted, extra cellular form of the soluble part of the (pro)renin receptor as set forth by SEQ ID NO:1 or fragments thereof, in a sample of a biological fluid previously collected in a patient or an animal.

9. *In vitro* process according to claim 8, for the diagnosis and/or the prognostic of neurodegenerative disease in a patient liable to be afflicted by a neurodegenerative disease, in particular Alzheimer's disease, dementia with Lewy bodies, fronto- temporal dementia, Parkinson's disease, amyotrophic lateral sclerosis, wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO: 1 or fragments thereof, as determined by an Elisa test, in a patient liable to be afflicted by a neurodegenerative disease;
b. *In vitro* determination of the level of secreted, extra cellular form of the soluble part of the (pro)renin receptor as set forth by SEQ ID NO:1 or fragments thereof, as determined by an Elisa test, in control patients or animals;
c. Comparison of both values, the value determined in step a. being higher than the one of step b. being indicative of a neurodegenerative disease or a risk to have a neurodegenerative disease.

10. *In vitro* process according to claim 8, for the follow up of a treatment against a neurodegenerative disease, in particular Alzheimer's disease, dementia with Lewy bodies, fronto-temporal dementia, Parkinson's disease, amyotrophic lateral sclerosis, wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO: 1 or fragments thereof, as determined by an Elisa test, in a patient or animal previously treated with said treatment,
b. *In vitro* determination of the level of the secreted, extra cellular form of the soluble part of the (pro)renin receptor as set forth by SEQ ID NO:1 or fragments thereof, as determined by an Elisa test, in said patient before said treatment;
c. Comparison of both values, the value determined in step a. being lower than the one of step b. being indicative of the efficacy of said treatment.

11. *In vitro* process according to claim 8, for the follow up of a treatment stimulating the neuroregeneration in a previously treated patient,
wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO: 1 or fragments thereof, as determined by an Elisa test, in a patient or animal previously treated with said treatment,
b. *In vitro* determination of the level of the secreted, extra cellular form of the soluble part of the (pro)renin receptor as set forth by SEQ ID NO: 1 or fragments thereof, as determined by an Elisa test, in control patients or animals;
c. Comparison of both values, the value determined in step a. being lower than the one of step b. being indicative of the neuroregeneration.

12. *In vitro* process according to claim 8, for the screening of new drugs liable to treat neurodegenerative pathologies in a patient or animal, in particular Alzheimer's disease, dementia with Lewy bodies, fronto-temporal dementia, Parkinson's disease, amyotrophic lateral sclerosis,
wherein said fluid is the cerebrospinal fluid, in particular the human cerebrospinal fluid,
comprising the following steps:
a. *In vitro* determination of the level of the secreted, extra cellular form of the soluble part of the (pro)renin receptor as set forth by SEQ ID NO: 1 or fragments thereof, as determined by an Elisa test, in a patient or animal afflicted by a neurodegenerative disease and previously treated with said new drug,
b. *In vitro* determination of the level of the secreted, extra cellular form of the soluble part of the (pro)renin receptor as set forth by SEQ ID NO: 1 or fragments thereof, as determined by an Elisa test, in a patient or animal afflicted by a neurodegenerative disease before treatment or in control patients or animals;
c. Comparison of both values, the value determined in step a. for said patient previously treated with said new drug being lower than the one determined in step b. or equal to the one of control patients or animals, being indicative of an efficacy of said new drug.

13. *In vitro* process according to claim 8, for the diagnosis and/or the prognosis of tumoral and/or proliferative disorders of the central nervous system, in particular glioblastomas,
wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO: 1 or fragments thereof, as determined by an Elisa test, in a patient liable to be afflicted by tumoral and/or proliferative disorders of the central nervous system, m particular glioblastomas;
b. *In vitro* determination of the level of secreted, extra cellular form of the soluble part of the (pro)renin receptor as set forth by SEQ ID NO:1 or fragments thereof,
as determined by an Elisa test, in control patients or animals;
c. Comparison of both values, the value determined in step a. being higher than the one of step b. being indicative of a tumoral and/or proliferative disorders of the central nervous system, in particular glioblastomas or a risk to have a tumoral and/or proliferative disorders of the central nervous system, in particular glioblastomas.

14. *In vitro* process according to claim 8, for the follow-up of tumors of the central nervous system after surgery and/or chemotherapy and/or radiotherapy,
wherein said fluid is the cerebrospinal fluid, in particular the human cerebrospinal fluid, comprising the following steps:
a. *In vitro* determination of the level of the secreted, extra cellular form of the soluble part of the (pro)renin receptor as set forth by SEQ ID NO:1 or fragments thereof, as determined by an Elisa test, in a patient having a tumoral and/or proliferative disorders of the central nervous system, in particular glioblastomas
and subjected to a surgery and/or chemotherapy and/or radiotherapy,
b. *In vitro* determination of the level of the secreted, extra cellular form of the soluble part of the (pro)renin receptor as set forth by SEQ ID NO: 1 or fragments thereof, as determined by an Elisa test, in a patient having a tumoral and/or proliferative disorders of the central nervous system, in particular glioblastomas
before said surgery and/or chemotherapy and/or radiotherapy,
c. Comparison of both values, the value determined in step a. being lower than the one of step b. being indicative of a the efficacy of surgery and/or chemotherapy and/or radiotherapy.

15. Use of a kit according to the uses and processes of any claims 1 to 14, the kit comprising an antibody specifically binding to one of the amino acid sequence set forth by SEQ ID 4-8 for the *in vitro* determination of the level of the secreted, extra cellular form of the soluble part of the (pro)renin receptor as set forth by SEQ ID NO:1 or fragments thereof, in a sample of a biological fluid previously collected in a patient or an animal, in particular in the cerebrospinal fluid, and means for the detection of the immune complex formed between said antibody and said secreted, extra cellular form of the soluble part of the (pro)renin receptor as set forth by SEQ ID NO:1 or fragment thereof.

## Patentansprüche

1. *In-vitro*-Verwendung der sezernierten, extrazellulären Form des löslichen Teils eines (Pro)reninzeptors, wie er von SEQ ID NO:1 angegeben wird, oder von Fragmenten davon, die an Renin oder Prorenin binden, deren Konzentration in einer Probe einer biologischen Flüssigkeit bestimmt wurde, die zuvor von einem menschlichen Patienten oder einem Tier genommen wurde, als präsymptomatischen dualen Biomarker für Neurodegeneration und Neuroregeneration, wobei die biologische Flüssigkeit Cerebrospinalflüssigkeit oder das extrazelluläre Medium irgendeiner neuronalen Zelle in Primärkultur ist, insbesondere in Kleinhirnkörnerzellen in Primärkultur, wobei die Konzentration in Bezug auf eine Probe der biologischen Flüssigkeit, die zuvor von einem menschlichen Kontrollpatienten oder einem Kontrolltier genommen wurde, moduliert ist.

2. Verwendung der Konzentration der sezernierten, extrazellulären Form des löslichen Teils des (Pro)reninzeptors, wie er von SEQ ID NO:1 angegeben wird, oder von Fragmenten davon, die an das Renin oder Prorenin binden, die *in vitro* in einer Probe einer zuvor von einem menschlichen Patienten oder einem Tier genommenen biologischen Flüssigkeit bestimmt worden ist, für die Diagnose und/oder die Prognose einer neurodegenerativen Krankheit oder eines Stadiums einer neurodegenerativen Krankheit in einem Patienten, von dem angenommen wird, dass er an einer neurodegenerativen Krankheit leidet, insbesondere an Alzheimer-Krankheit, Demenz mit Lewy-Körperchen, frontotemporaler Demenz, Parkinson-Krankheit, amyotropher Lateralsklerose, und/oder
für die Verlaufsuntersuchung im Zuge einer Behandlung gegen eine neurodegenerative Krankheit an einem Patienten, der an einer neurodegenerativen Krankheit leidet, insbesondere an Alzheimer-Krankheit, Demenz mit Lewy-Körperchen, frontotemporaler Demenz, Parkinson-Krankheit, amyotropher Lateralsklerose, und/oder für die Verlaufsuntersuchung im Zuge einer Behandlung, durch welche die Neuroregeneration stimuliert wird, und/oder für die Auslese von neuen Arzneistoffen, die für die Behandlung neurodegenerativer Pathologien in Frage kommen, insbesondere von Alzheimer-Krankheit, Demenz mit Lewy-Körperchen, frontotemporaler Demenz, Parkinson-Krankheit, amyotropher Lateralsklerose, und/oder
für die Diagnose und/oder die Prognose tumoraler und/oder proliferativer Krankheiten des zentralen Nervensystems, insbesondere von Glioblastomen, und/oder
für die Verlaufsuntersuchung im Zuge einer Behandlung gegen tumorale und/oder proliferative Krankheiten des zentralen Nervensystems, insbesondere von Glioblastomen, nach einer Operation und/oder Chemotherapie und/oder Strahlentherapie,
wobei die Konzentration in Bezug auf eine Probe der biologischen Flüssigkeit, die zuvor von einem Kontrollpatienten oder einem Kontrolltier genommen wurde, moduliert ist.

3. Verwendung nach Anspruch 2 für die Diagnose und/oder die Prognose einer neurodegenerativen Krankheit oder eines Stadiums einer neurodegenerativen Krankheit, insbesondere von Alzheimer-Krankheit, Demenz mit Lewy-Körperchen, frontotemporaler Demenz, Parkinson-Krankheit, amyotropher Lateralsklerose,
wobei die Flüssigkeit die Cerebrospinalflüssigkeit, insbesondere die menschliche Cerebrospinalflüssigkeit ist, wobei die Konzentration der sezernierten, extrazellulären Form des löslichen Teils des (Pro)reninzeptors, wie er von SEQ ID NO:1 angegeben wird, gemäß einer Bestimmung durch einen ELISA-Test höher ist als diejenige, die an Kontrollpatienten oder - tieren bestimmt wird, höher als 42 ng/ml, insbesondere höher als 50 ng/ml, wie an Kontrollpatienten bestimmt.

4. Verwendung nach Anspruch 2
für die Verlaufsuntersuchung im Zuge einer Behandlung einer neurodegenerativen Krankheit an einem menschlichen Patienten oder einem Tier, insbesondere von Alzheimer-Krankheit, Demenz mit Lewy-Körperchen, frontotemporaler Demenz, Parkinson-Krankheit, amyotropher Lateralsklerose,
wobei die Flüssigkeit die Cerebrospinalflüssigkeit, insbesondere die menschliche Cerebrospinalflüssigkeit ist, wobei die Konzentration der sezernierten, extrazellulären Form des löslichen Teils des (Pro)reninzeptors, wie er von SEQ ID NO:1 angegeben wird, gemäß einer Bestimmung durch einen ELISA-Test in einem Patienten oder einem Tier, der bzw. das zuvor mit der Behandlung behandelt wurde, niedriger ist als diejenige, die an dem Patienten oder dem Tier vor der Behandlung bestimmt wurde,
oder für die Verlaufsuntersuchung im Zuge der Behandlung, welche die Neuroregeneration in einem menschlichen Patienten oder einem Tier stimuliert, wobei die Flüssigkeit die Cerebrospinalflüssigkeit, insbesondere die menschliche Cerebrospinalflüssigkeit ist, wobei die Konzentration der sezernierten, extrazellulären Form des löslichen Teils des (Pro)reninzeptors, wie er von SEQ ID NO:1 angegeben wird, gemäß einer Bestimmung durch einen ELISA-Test in einem Patienten oder einem Tier, der bzw. das zuvor mit der Behandlung behandelt wurde, niedriger ist als 50 ng/ml.

5. Verwendung nach Anspruch 2 für die Auslese von neuen Arzneistoffen, die für die Behandlung von neurodegenerativen Krankheiten in einem menschlichen Patienten oder einem Tier in Frage kommen, insbesondere von Alzheimer-Krankheit, Demenz mit Lewy-Körperchen, frontotemporaler Demenz, Parkinson-Krankheit, amyotropher Lateralsklerose,
wobei die Flüssigkeit die Cerebrospinalflüssigkeit, insbesondere die menschliche Cerebrospinalflüssigkeit ist, wobei die Konzentration der sezernierten, extrazellulären Form des löslichen Teils des (Pro)reninzeptors, wie er von SEQ ID NO:1 angegeben wird, wie gemäß einem ELISA-Test bestimmt, in einem Patienten oder einem Tier, der bzw. das an einer neurodegenerativen Krankheit leidet und der bzw. das zuvor mit dem neuen Arzneistoff behandelt wurde, niedriger ist als diejenige, die in dem Patienten oder dem Tier vor der Behandlung mit dem neuen Arzneistoff bestimmt wurde, oder als diejenige, die an Kontrollpatienten bestimmt wird.

6. Verwendung nach Anspruch 2 für die Diagnose und/oder die Prognose von tumoralen und/oder proliferativen Krankheiten des zentralen Nervensystems, insbesondere von Glioblastomen,
wobei die Flüssigkeit die Cerebrospinalflüssigkeit, insbesondere die menschliche Cerebrospinalflüssigkeit ist, wobei die Konzentration der sezernierten, extrazellulären Form des löslichen Teils des (Pro)reninzeptors, wie er von SEQ ID NO:1 angegeben wird, wie durch einen ELISA-Test bestimmt, höher ist als 42 ng/ml, insbesondere höher ist als 50 ng/ml, wie sie an Kontrollpatienten oder -tieren bestimmt wird.

7. Verwendung nach Anspruch 2 für die Verlaufsuntersuchung von Tumoren des zentralen Nervensystems nach einer Operation und/oder Chemotherapie und/oder Strahlentherapie,
wobei die Flüssigkeit die Cerebrospinalflüssigkeit, insbesondere die menschliche Cerebrospinalflüssigkeit ist, wobei die Konzentration der sezernierten, extrazellulären Form des löslichen Teils des (Pro)reninzeptors, wie er von SEQ ID NO:1 angegeben wird, wie durch einen ELISA-Test bestimmt, in einem Patienten, der einer Operation und/oder Chemotherapie unterzogen wurde, höher ist als diejenige, die an dem Patienten oder dem Tier vor der Operation und/oder Chemotherapie bestimmt wurde, oder derjenigen gleich ist, die an Kontrollpatienten bestimmt wird.

8. *In-vitro*-Verfahren für die *in-vitro*-Diagnose und/oder die Prognose einer neurodegenerativen Krankheit oder eines Stadiums einer neurodegenerativen Krankheit an einem Patienten, von dem angenommen wird, dass er an einer neurodegenerativen Krankheit leidet, insbesondere an Alzheimer-Krankheit, Demenz mit Lewy-Körperchen, frontotemporaler Demenz, Parkinson-Krankheit, amyotropher Lateralsklerose, und/oder
für die Verlaufsuntersuchung im Zuge einer Behandlung gegen eine neurodegenerative Krankheit an einem Patienten, der an einer neurodegenerativen Krankheit leidet, insbesondere an Alzheimer-Krankheit, Demenz mit Lewy-Körperchen, frontotemporaler Demenz, Parkinson-Krankheit, amyotropher Lateralsklerose, und/oder
für die Verlaufsuntersuchung Zuge einer Behandlung zur Stimulierung der Neuroregeneration und/oder
für die Auslese neuer Arzneistoffe, die für die Behandlung neurodegenerativer Pathologien in Frage kommen, insbesondere von Alzheimer-Krankheit, Demenz mit Lewy-Körperchen, frontotemporaler Demenz, Parkinson-Krankheit, amyotropher Lateralsklerose, und/oder
für die Diagnose und/oder die Prognose von tumoralen und/oder proliferativen Krankheiten des zentralen Nervensystems, insbesondere Glioblastomen, und/oder
für die Verlaufsuntersuchung im Zuge einer Behandlung von tumoralen und/oder proliferativen Krankheiten des zentralen Nervensystems insbesondere Glioblastomen, nach einer Operation und/oder Chemotherapie und/oder Strahlentherapie,
das Bestimmen der Konzentration der sezernierten, extrazellulären Form des löslichen Teils des (Pro)reninzeptors, wie er von SEQ ID NO:1 angegeben wird, oder von Fragmenten davon in einer Probe einer biologischen Flüssigkeit, die zuvor von einem Patienten oder einem Tier genommen wurde, *in vitro* umfassend.

9. *In-vitro*-Verfahren nach Anspruch 8 für die Diagnose und/oder die Prognose einer neurodegenerativen Krankheit an einem Patienten, von dem angenommen wird, dass er an einer neurodegenerativen Krankheit leidet, insbesondere an Alzheimer-Krankheit, Demenz mit Lewy-Körperchen, frontotemporaler Demenz, Parkinson-Krankheit, amyotropher Lateralsklerose, wobei die Flüssigkeit die Cerebrospinalflüssigkeit, insbesondere die menschliche Cerebrospinalflüssigkeit ist,
die folgenden Schritte umfassend:
a. Bestimmen der Konzentration der sezernierten, extrazellulären Form des löslichen Teils des (Pro)reninzeptors, wie er von SEQ ID NO:1 angegeben wird, oder von Fragmenten davon an einem Patienten, von dem angenommen wird, dass er an einer neurodegenerativen Krankheit leidet, *in vitro*, bestimmt durch einen ELISA-Test;
b. Bestimmen der Konzentration der sezernierten, extrazellulären Form des löslichen Teils des (Pro)reninzeptors, wie er von SEQ ID NO:1 angegeben wird, oder von Fragmenten davon an Kontrollpatienten oder -tieren, *in vitro*;
c. Vergleichen beider Werte, wobei der in Schritt a. bestimmte Wert, wenn er höher ist als derjenige, der in Schritt b. bestimmt wird, einen Hinweis auf eine neurodegenerative Krankheit oder ein Risiko für eine neurodegenerative Krankheit liefert.

10. *In-vitro*-Verfahren nach Anspruch 8 für die Verlaufsuntersuchung im Zuge einer Behandlung einer neurodegenerativen Krankheit, insbesondere von Alzheimer-Krankheit, Demenz mit Lewy-Körperchen, frontotemporaler Demenz, Parkinson-Krankheit, amyotropher Lateralsklerose,
wobei die Flüssigkeit die Cerebrospinalflüssigkeit, insbesondere die menschliche Cerebrospinalflüssigkeit ist,
die folgenden Schritte umfassend:
a. Bestimmen der Konzentration der sezernierten, extrazellulären Form des löslichen Teils des (Pro)reninzeptors, wie er von SEQ ID NO:1 angegeben wird, oder von Fragmenten davon an einem Patienten oder einem Tier, der bzw. das zuvor mit der Behandlung behandelt wurde, *in vitro,* bestimmt durch einen ELISA-Test,
b. Bestimmen der Konzentration der sezernierten, extrazellulären Form des löslichen Teils des (Pro)reninzeptors, wie er von SEQ ID NO:1 angegeben wird, oder von Fragmenten davon an dem Patienten vor der Behandlung, *in vitro,* bestimmt durch einen ELISA-Test;
c. Vergleichen beider Werte, wobei der in Schritt a. bestimmte Wert, wenn er niedriger ist als derjenige von Schritt b, einen Hinweis auf die Wirksamkeit der Behandlung liefert.

11. *In-vitro*-Verfahren nach Anspruch 8 für die Verlaufsuntersuchung im Zuge einer Behandlung, mit der die Neuroregeneration in einem zuvor behandelten Patienten stimuliert wird, wobei die Flüssigkeit die Cerebrospinalflüssigkeit, insbesondere die menschliche Cerebrospinalflüssigkeit ist, die folgenden Schritte umfassend:
a. Bestimmen der Konzentration der sezernierten, extrazellulären Form des löslichen Teils des (Pro)reninzeptors, wie er von SEQ ID NO:1 angegeben wird, oder von Fragmenten davon an einem Patienten oder einem Tier, der bzw. das zuvor mit der Behandlung behandelt wurde, *in vitro,* bestimmt durch einen ELISA-Test,
b. Bestimmen der Konzentration der sezernierten, extrazellulären Form des löslichen Teils des (Pro)reninzeptors, wie er von SEQ ID NO:1 angegeben wird, oder von Fragmenten davon an Kontrollpatienten oder -tieren, *in vitro,* bestimmt durch einen ELISA-Test;
c. Vergleichen beider Werte, wobei der in Schritt a. bestimmte Wert, wenn er niedriger ist als derjenige von Schritt b., einen Hinweis auf die Neuroregeneration liefert.

12. *In-vitro*-Verfahren nach Anspruch 8 für die Auslese von neuen Arzneistoffen, die für die Behandlung von neurodegenerativen Krankheiten an einem Patienten oder einem Tier in Frage kommen, insbesondere von Alzheimer-Krankheit, Demenz mit Lewy-Körperchen, frontotemporaler Demenz, Parkinson-Krankheit, amyotropher Lateralsklerose,
wobei die Flüssigkeit die Cerebrospinalflüssigkeit, insbesondere die menschliche Cerebrospinalflüssigkeit ist,
die folgenden Schritte umfassend:
a. Bestimmen der Konzentration der sezernierten, extrazellulären Form des löslichen Teils des (Pro)reninzeptors, wie er von SEQ ID NO:1 angegeben wird, oder von Fragmenten davon an einem Patienten oder einem Tier, der bzw. das an einer neurodegenerativen Krankheit leidet und der bzw. das zuvor mit dem neuen Arzneistoff behandelt wurde, *in vitro,* bestimmt durch einen ELISA-Test
b. Bestimmen der Konzentration der sezernierten, extrazellulären Form des löslichen Teils des (Pro)reninzeptors, wie er von SEQ ID NO:1 angegeben wird, oder von Fragmenten davon an einem Patienten oder einem Tier, der bzw. das an einer neurodegenerativen Krankheit leidet, vor der Behandlung oder an Kontrollpatienten oder -tieren *in vitro,* bestimmt durch einen ELISA-Test;
c. Vergleichen beider Werte, wobei der Wert, der in Schritt a. für den Patienten bestimmt wird, der zuvor mit dem neuen Arzneistoff behandelt wurde, wenn er niedriger ist als derjenige, der in Schritt b. bestimmt wird, oder dem von Kontrollpatienten oder -tieren gleich ist, einen Hinweis auf eine Wirksamkeit des neuen Arzneistoffs liefert.

13. *In-vitro*-Verfahren nach Anspruch 8 für die Diagnose und/oder die Prognose von tumoralen und/oder proliferativen Krankheiten des zentralen Nervensystems, insbesondere Glioblastomen,
wobei die Flüssigkeit die Cerebrospinalflüssigkeit, insbesondere die menschliche Cerebrospinalflüssigkeit ist, die folgenden Schritte umfassend:
a. Bestimmen der Konzentration der sezernierten, extrazellulären Form des löslichen Teils des (Pro)reninzeptors, wie er von SEQ ID NO:1 angegeben wird, oder von Fragmenten davon an einem Patienten, von dem angenommen wird, dass er an tumoralen und/oder proliferativen Krankheiten des zentralen Nervensystems, insbesondere Glioblastomen, leidet, *in vitro,* bestimmt durch einen ELISA-Test;
b. Bestimmen der Konzentration der sezernierten, extrazellulären Form des löslichen Teils des (Pro)reninzeptors, wie er von SEQ ID NO:1 angegeben wird, oder von Fragmenten davon an Kontrollpatienten oder -tieren *in vitro,* bestimmt durch einen ELISA-Test;
c. Vergleichen beider Werte, wobei der in Schritt a. bestimmte Wert, wenn er höher ist als derjenige in Schritt b., einen Hinweis auf tumorale und/oder proliferative Krankheiten des zentralen Nervensystems, insbesondere auf Glioblastome, oder auf ein Risiko für tumorale und/oder proliferative Störungen des zentralen Nervensystems, insbesondere auf Glioblastome, liefert.

14. *In-vitro*-Verfahren nach Anspruch 8 für die Verlaufsuntersuchung von Tumoren des zentralen Nervensystems nach einer Operation und/oder Chemotherapie und/oder Strahlentherapie,
wobei die Flüssigkeit die Cerebrospinalflüssigkeit, insbesondere die menschliche Cerebrospinalflüssigkeit ist,
die folgenden Schritte umfassend:
a. Bestimmen der Konzentration der sezernierten, extrazellulären Form des löslichen Teils des (Pro)reninzeptors, wie er von SEQ ID NO:1 angegeben wird, oder von Fragmenten davon an einem Patienten mit tumoralen und/oder proliferativen Krankheiten des zentralen Nervensystems, insbesondere Glioblastomen, der einer Operation und/oder Chemotherapie und/oder Strahlentherapie unterzogen wurde, *in vitro,* bestimmt durch einen ELISA-Test,
b. Bestimmen der Konzentration der sezernierten, extrazellulären Form des löslichen Teils des (Pro)reninzeptors, wie er von SEQ ID NO:1 angegeben wird, oder von Fragmenten davon an einem Patienten mit tumoralen und/oder proliferativen Krankheiten des zentralen Nervensystems, insbesondere Glioblastomen, vor der Operation und/oder Chemotherapie und/oder Strahlentherapie, *in vitro,* bestimmt durch einen ELISA-Test,
c. Vergleichen beider Werte, wobei der in Schritt a. bestimmte Wert, wenn er niedriger ist als derjenige von Schritt b., einen Hinweis auf die Wirksamkeit einer Operation und/oder Chemotherapie und/oder Strahlentherapie liefert.

15. Verwendung eines Kits für die Verwendungen und Verfahren nach einem der Ansprüche 1 bis 14, wobei das Kit umfasst: einen Antikörper, der spezifisch an eine der Aminosäuresequenzen bindet, die von SEQ ID 4-8 angegeben werden, für die *in-vitro-*Bestimmung der Konzentration der sezernierten, extrazellulären Form des löslichen Teils des (Pro)reninzeptors, wie er von SEQ ID NO:1 angegeben wird, oder von Fragmenten davon in einer Probe einer biologischen Flüssigkeit, die zuvor von einem Patienten oder einem Tier gewonnen wurde, insbesondere in der Cerebrospinalflüssigkeit, und eine Einrichtung zum Nachweisen des Immunkomplexes, der zwischen dem Antikörper und der sezernierten, extrazellulären Form des löslichen Teils des (Pro)reninzeptors, wie er von SEQ ID NO:1 angegeben wird, oder von Fragmenten davon gebildet wird.

## Revendications

1. Utilisation *in vitro* de la forme extracellulaire sécrétée de la partie soluble du récepteur de (pro)rénine telle que définie par SEQ ID NO: 1 ou des fragments de celle-ci qui se lient à la rénine ou à la pro-rénine, dont le niveau a été déterminé dans un échantillon d'un liquide biologique prélevé préalablement chez un patient humain ou un animal, en tant que biomarqueur pré-symptomatique à la fois de neurodégénérescence et à la fois de neurorégénération, ledit liquide biologique étant le liquide céphalorachidien ou le milieu extracellulaire de toute cellule neuronale en culture primaire, en particulier dans des neurones granulaires cérébelleux en culture primaire, ledit niveau étant modulé par rapport à un échantillon dudit liquide biologique recueilli préalablement chez un patient humain témoin ou un animal témoin.

2. Utilisation du niveau de la forme extracellulaire sécrétée de la partie soluble du récepteur de (pro)rénine telle que définie par SEQ ID NO: 1 ou des fragments de celle-ci qui se lient à la rénine ou à la pro-rénine, qui a été déterminée *in vitro* dans un échantillon d'un liquide biologique recueilli préalablement chez un patient humain ou un animal, pour le diagnostic et/ou le pronostic d'une maladie neurodégénérative ou d'un stade de maladie neurodégénérative chez un patient susceptible d'être atteint d'une maladie neurodégénérative, en particulier la maladie d'Alzheimer, la démence à corps de Lewy, la démence frontotemporale, la maladie de Parkinson, la sclérose latérale amyotrophique et/ou pour le suivi d'un traitement contre une maladie neurodégénérative chez un patient atteint d'une maladie neurodégénérative, en particulier la maladie d'Alzheimer, la démence à corps de Lewy, la démence frontotemporale, la maladie de Parkinson, la sclérose latérale amyotrophique et/ou pour le suivi d'un traitement stimulant la neurorégénération et/ou pour le criblage de nouveaux médicaments susceptibles de traiter les pathologies neurodégénératives, en particulier la maladie d'Alzheimer, la démence à corps de Lewy, la démence frontotemporale, la maladie de Parkinson, la sclérose latérale amyotrophique et/ou pour le diagnostic et/ou le pronostic de troubles tumoraux et/ou prolifératifs du système nerveux central, en particulier des glioblastomes et/ou pour le suivi d'un traitement contre les troubles tumoraux et/ou prolifératifs du système nerveux central, en particulier des glioblastomes, après une chirurgie et/ou une chimiothérapie et/ou une radiothérapie, ledit niveau étant modulé par rapport à un échantillon dudit liquide biologique recueilli préalablement chez un patient témoin ou un animal témoin.

3. Utilisation selon la revendication 2, dans le diagnostic et/ou le pronostic d'une maladie neurodégénérative ou d'un stade de maladie neurodégénérative, en particulier la maladie d'Alzheimer, la démence à corps de Lewy, la démence fronto-temporale, la maladie de Parkinson, la sclérose latérale amyotrophique, ledit liquide étant le liquide céphalorachidien, en particulier le liquide céphalorachidien humain, dans lequel le niveau de la forme extracellulaire sécrétée de la partie soluble du récepteur de (pro)rénine telle que définie par SEQ ID NO: 1, tel que déterminé par un test Elisa, est supérieur à celui des patients ou des animaux témoins, supérieur à 42 ng/ml, en particulier supérieur à 50 ng/ml, tel que déterminé chez les patients témoins.

4. Utilisation selon la revendication 2, dans le suivi d'un traitement contre une maladie neurodégénérative chez un patient humain ou un animal, en particulier la maladie d'Alzheimer, la démence à corps de Lewy, la démence frontotemporale, la maladie de Parkinson, la sclérose latérale amyotrophique,
ledit liquide étant le liquide céphalorachidien, en particulier le liquide céphalorachidien humain, dans lequel le niveau de la forme extracellulaire sécrétée de la partie soluble du récepteur de (pro)rénine telle que définie par SEQ ID NO: 1, tel que déterminé par un test Elisa, chez un patient ou un animal préalablement traité avec ledit traitement, est inférieur à celui déterminé chez ledit patient ou animal avant ledit traitement,
ou dans le suivi du traitement stimulant la neurorégénération chez un patient humain ou animal, ledit liquide étant le liquide céphalorachidien, en particulier le liquide céphalorachidien humain, dans lequel le niveau de la forme extracellulaire sécrétée de la partie soluble du récepteur de (pro)rénine telle que définie par SEQ ID NO: 1, tel que déterminé par un test Elisa, chez un patient ou un animal préalablement traité avec ledit traitement, est inférieur à 50 ng/ml.

5. Utilisation selon la revendication 2, dans le criblage de nouveaux médicaments susceptibles de traiter des pathologies neurodégénératives chez un patient humain ou un animal, en particulier la maladie d'Alzheimer, la démence à corps de Lewy, la démence frontotemporale, la maladie de Parkinson, la sclérose latérale amyotrophique,
ledit liquide étant le liquide céphalorachidien, en particulier le liquide céphalorachidien humain, dans lequel le niveau de la forme extracellulaire sécrétée de la partie soluble du récepteur de (pro)rénine telle que définie par SEQ ID NO: 1, tel que déterminé par un test Elisa, chez un patient ou un animal atteint d'une maladie neurodégénérative, préalablement traité avec ledit nouveau médicament, est plus faible que celui déterminé chez ledit patient ou animal avant le traitement avec ledit nouveau médicament, ou égal à celui déterminé chez les patients témoins.

6. Utilisation selon la revendication 2, dans le diagnostic et/ou le pronostic de troubles tumoraux et/ou prolifératifs du système nerveux central, en particulier des glioblastomes,
ledit liquide étant le liquide céphalorachidien, en particulier le liquide céphalorachidien humain, dans lequel le niveau de la forme extracellulaire sécrétée de la partie soluble du récepteur de (pro)rénine telle que définie par SEQ ID NO: 1, tel que déterminé par un test ELISA, est supérieur à 42 ng/ml, en particulier supérieur à 50 ng/ml, tel que déterminé chez les patients ou les animaux témoins.

7. Utilisation selon la revendication 2, dans le suivi de tumeurs du système nerveux central après une chirurgie et/ou une chimiothérapie et/ou une radiothérapie,
ledit liquide étant le liquide céphalorachidien, en particulier le liquide céphalorachidien humain, dans lequel le niveau de la forme extracellulaire sécrétée de la partie soluble du récepteur de (pro)rénine telle que définie par SEQ ID NO: 1, tel que déterminé par un test Elisa, chez un patient ayant subi une chirurgie et/ou une chimiothérapie est supérieur à celui déterminé chez ledit patient ou animal avant la chirurgie et/ou la chimiothérapie, ou égal à celui déterminé les patients témoins.

8. Procédé *in vitro* pour le diagnostic *in vitro* et/ou le pronostic d'une maladie neurodégénérative ou d'un stade de maladie neurodégénérative chez un patient atteint d'une maladie neurodégénérative, en particulier la maladie d'Alzheimer, la démence à corps de Lewy, la démence frontotemporale, la maladie de Parkinson, la sclérose latérale amyotrophique et/ou pour le suivi d'un traitement contre une maladie neurodégénérative chez un patient atteint d'une maladie neurodégénérative, en particulier la maladie d'Alzheimer, la démence à corps de Lewy, la démence fronto-temporale, la maladie de Parkinson, la sclérose latérale amyotrophique et/ou
pour le suivi d'un traitement stimulant la neurorégénération. et/ou
pour le criblage de nouveaux médicaments susceptibles de traiter des pathologies neurodégénératives, en particulier la maladie d'Alzheimer, la démence à corps de Lewy, la démence frontotemporale, la maladie de Parkinson, la sclérose latérale amyotrophique et/ou
pour le diagnostic et/ou le pronostic de troubles tumoraux et/ou prolifératifs du système nerveux central, en particulier des glioblastomes et/ou
pour le suivi d'un traitement contre les troubles tumoraux et/ou prolifératifs du système nerveux central, en particulier des glioblastomes, après une chirurgie et/ou une chimiothérapie et/ou radiothérapie,
comprenant la détermination *in vitro* du niveau de la forme extracellulaire sécrétée de la partie soluble du récepteur de (pro)rénine telle que définie par SEQ ID NO: 1 ou des fragments de celle-ci, dans un échantillon d'un liquide biologique recueilli préalablement chez un patient ou un animal.

9. Procédé *in vitro* selon la revendication 8, pour le diagnostic et/ou le pronostic d'un maladie neurodégénérative chez un patient susceptible d'être atteint d'une maladie neurodégénérative, en particulier la maladie d'Alzheimer, la démence à corps de Lewy, la démence frontotemporale, la maladie de Parkinson, la sclérose latérale amyotrophique,
ledit liquide étant le liquide céphalo-rachidien, en particulier le liquide céphalorachidien humain,
comprenant les étapes suivantes :
a. la détermination *in vitro* du niveau de la forme extracellulaire sécrétée de la partie soluble du récepteur de (pro)rénine telle que défini par SEQ ID NO: 1 ou des fragments de celle-ci, tel que déterminé par un test ELISA, chez un patient susceptible d'être atteint d'une maladie neurodégénérative ;
b. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez des patients ou des animaux témoins ;
c. la comparaison des deux valeurs, la valeur déterminée à l'étape a. étant supérieure à celle de l'étape b, et étant indicative d'une maladie neurodégénérative ou d'un risque d'avoir une maladie neurodégénérative.

10. Procédé *in vitro* selon la revendication 8, destiné au suivi d'un traitement contre une maladie neurodégénérative, en particulier la maladie d'Alzheimer, la démence à corps de Lewy, la démence frontotemporale, la maladie de Parkinson, la sclérose latérale amyotrophique,
ledit liquide étant le liquide céphalorachidien, en particulier le liquide céphalorachidien humain,
comprenant les étapes suivantes :
a. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez un patient ou un animal préalablement traité avec ledit traitement,
b. la détermination *in vitro* du niveau de la forme extracellulaire sécrétée par la partie soluble du récepteur de (pro)rénine telle que définie par SEQ ID NO: 1 ou des fragments de celle-ci, tel que déterminé par un test Elisa, chez ledit patient avant ledit traitement ;
c. la comparaison des deux valeurs, la valeur déterminée à l'étape a. étant inférieure à celle de l'étape b. et étant indicative de l'efficacité dudit traitement.

11. Procédé *in vitro* selon la revendication 8, destiné au suivi d'un traitement stimulant la neurorégénération chez un patient préalablement traité,
ledit liquide étant le liquide céphalorachidien, en particulier le liquide céphalorachidien humain,
comprenant les étapes suivantes :
a. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez un patient ou un animal préalablement traité avec ledit traitement ;
b. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez des patients ou des animaux témoins ;
c. la comparaison des deux valeurs, la valeur déterminée à l'étape a. étant inférieure à celle de l'étape b. et étant indicative de la neurorégénération.

12. Procédé *in vitro* selon la revendication 8, destiné au criblage de nouveaux médicaments susceptibles de traiter des pathologies neurodégénératives chez un patient ou un animal, en particulier la maladie d'Alzheimer, la démence à corps de Lewy, la démence fronto-temporale, la maladie de Parkinson, la sclérose latérale amyotrophique,
ledit liquide étant le liquide céphalorachidien, en particulier le liquide céphalorachidien humain, comprenant les étapes suivantes :
a. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez un patient ou un animal atteint d'une maladie neurodégénérative et traité préalablement avec ce nouveau médicament ;
b. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez un patient ou un animal atteint d'une maladie neurodégénérative avant le traitement ou chez des patients ou des animaux témoins ;
c. la comparaison des deux valeurs, la valeur déterminée à l'étape a. pour ledit patient préalablement traité avec ledit nouveau médicament étant inférieure à celle déterminée à l'étape b. ou égale à celle des patients ou des animaux témoins, et étant indicative de l'efficacité de ce nouveau médicament.

13. Procédé *in vitro* selon la revendication 8, destiné au diagnostic et/ou au pronostic de troubles tumoraux et/ou prolifératifs du système nerveux central, en particulier des glioblastomes,
ledit liquide étant le liquide céphalo-rachidien, en particulier le liquide céphalo-rachidien humain,
comprenant les étapes suivantes :
a. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez un patient susceptible d'être atteint de troubles tumoraux et/ou prolifératifs du système nerveux central, en particulier des glioblastomes ;
b. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez des patients ou des animaux témoins ;
c. la comparaison des deux valeurs, la valeur déterminée à l'étape a. étant supérieure à celle de l'étape b. et étant indicative d'un trouble tumoral et/ou prolifératif du système nerveux central, en particulier des glioblastomes ou d'un risque d'avoir un trouble tumoral et/ou prolifératif du système nerveux central, en particulier des glioblastomes.

14. Procédé *in vitro* selon la revendication 8, destiné au suivi de tumeurs du système nerveux central après une chirurgie et/ou une chimiothérapie et/ou une radiothérapie,
ledit liquide étant le liquide céphalorachidien, en particulier le liquide céphalorachidien humain,
comprenant les étapes suivantes :
a. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez un patient ayant un trouble tumoral et/ou prolifératif du système nerveux central, en particulier des glioblastomes et ayant subi une chirurgie et/ou une chimiothérapie et/ou une radiothérapie,
b. la détermination *in vitro* du niveau de la forme extracellulaire sécrétée de la partie soluble du récepteur de (pro)rénine telle que définie par SEQ ID NO: 1 ou des fragments de celle-ci, tel que déterminé par un test Elisa, chez un patient ayant un trouble tumoral et/ou prolifératif du système nerveux central, en particulier des glioblastomes, avant ladite chirurgie et/ou chimiothérapie et/ou radiothérapie ;
c. la comparaison des deux valeurs, la valeur déterminée à l'étape a. étant inférieure à celle de l'étape b. et étant indicative de l'efficacité de la chirurgie et/ou de la chimiothérapie et/ou de la radiothérapie ;

15. Utilisation d'un kit selon les utilisations et les procédés selon l'une quelconque des revendications 1 à 14, le kit comprenant un anticorps se liant spécifiquement à l'une des séquences d'acides aminés telles que définies par SEQ ID 4-8 pour la détermination *in vitro* du niveau de la forme extracellulaire sécrétée de la partie soluble du récepteur de (pro)rénine telle que définie par SEQ ID NO: 1 ou des fragments de celle-ci, dans un échantillon d'un liquide biologique recueilli préalablement chez un patient ou un animal, en particulier dans le liquide céphalo-rachidien, et des moyens de détection du complexe immune formé entre ledit anticorps et ladite forme extracellulaire sécrétée de la partie soluble du récepteur de (pro)rénine telle que définie par SEQ ID NO: 1 ou un fragment de celle-ci.


### SEQUENCE LISTING

<110> CNRS
   UPMC
   ENS
   COLLEGE DE FRANCE
   INSERM
<120> New dual biomarker of neurodegeneration and of neuroregeneration
<130> WOB 11 AD CNR NINE2
<150> EP 12 305 127.8
   <151> 2012-02-22
<150> EP 12 306 122.8
   <151> 2012-09-18
<160> 8
<170> PatentIn version 3.5
<210> 1
   <211> 262
   <212> PRT
   <213> Homo sapiens
<220>
   <221> PEPTIDE
   <222> (1) .. (262)
<400> 1
   Asn Glu Phe Ser Ile Leu Lys Ser Pro Gly Ser Val Val Phe Arg Asn
   1               5                   10                  15
   Gly Asn Trp Pro Ile Pro Gly Glu Arg Ile Pro Asp Val Ala Ala Leu
               20                  25                  30
   Ser Met Gly Phe Ser Val Lys Glu Asp Leu Ser Trp Pro Gly Leu Ala
           35                  40                  45
   Val Gly Asn Leu Phe His Arg Pro Arg Ala Thr Val Met Val Met Val
       50                  55                  60
   Lys Gly Val Asn Lys Leu Ala Leu Pro Pro Gly Ser Val Ile Ser Tyr
   65                  70                  75                  80
   Pro Leu Glu Asn Ala Val Pro Phe Ser Leu Asp Ser Val Ala Asn Ser
                   85                  90                  95
   Ile His Ser Leu Phe Ser Glu Glu Thr Pro Val Val Leu Gln Leu Ala
               100                 105                 110
   Pro Ser Glu Glu Arg Val Tyr Met Val Gly Lys Ala Asn Ser Val Phe
               115                 120                 125
   Glu Asp Leu Ser Val Thr Leu Arg Gln Leu Arg Asn Arg Leu Phe Gln
       130                 135                 140
   Glu Asn Ser Val Leu Ser Ser Leu Pro Leu Asn Ser Leu Ser Arg Asn
   145                 150                 155                 160
   Asn Glu Val Asp Leu Leu Phe Leu Ser Glu Leu Gln Val Leu His Asp
                   165                 170                 175
   Ile Ser Ser Leu Leu Ser Arg His Lys His Leu Ala Lys Asp His Ser
               180                 185                 190
   Pro Asp Leu Tyr Ser Leu Glu Leu Ala Gly Leu Asp Glu Ile Gly Lys
           195                 200                 205
   Arg Tyr Gly Glu Asp Ser Glu Gln Phe Arg Asp Ala Ser Lys Ile Leu
       210                 215                 220
   Val Asp Ala Leu Gln Lys Phe Ala Asp Asp Met Tyr Ser Leu Tyr Gly
   225                 230                 235                 240
   Gly Asn Ala Val Val Glu Leu Val Thr Val Lys Ser Phe Asp Thr Ser
                   245                 250                 255
   Leu Ile Arg Lys Thr Arg
               260
<210> 2
   <211> 293
   <212> PRT
   <213> Homo sapiens
<220>
   <221> PEPTIDE
   <222> (1)..(293)
<400> 2
   Asn Glu Phe Ser Ile Leu Lys Ser Pro Gly Ser Val Val Phe Arg Asn
   1               5                   10                  15
   Gly Asn Trp Pro Ile Pro Gly Glu Arg Ile Pro Asp Val Ala Ala Leu
               20                  25                  30
   Ser Met Gly Phe Ser Val Lys Glu Asp Leu Ser Trp Pro Gly Leu Ala
           35                  40                  45
   Val Gly Asn Leu Phe His Arg Pro Arg Ala Thr Val Met Val Met Val
       50                  55                  60
   Lys Gly Val Asn Lys Leu Ala Leu Pro Pro Gly Ser Val Ile Ser Tyr
   65                  70                  75                  80
   Pro Leu Glu Asn Ala Val Pro Phe Ser Leu Asp Ser Val Ala Asn Ser
                   85                  90                  95
   Ile His Ser Leu Phe Ser Glu Glu Thr Pro Val Val Leu Gln Leu Ala
               100                 105                 110
   Pro Ser Glu Glu Arg Val Tyr Met Val Gly Lys Ala Asn Ser Val Phe
           115                 120                 125
   Glu Asp Leu Ser Val Thr Leu Arg Gln Leu Arg Asn Arg Leu Phe Gln
       130                 135                 140
   Glu Asn Ser Val Leu Ser Ser Leu Pro Leu Asn Ser Leu Ser Arg Asn
   145                 150                 155                 160
   Asn Glu Val Asp Leu Leu Phe Leu Ser Glu Leu Gln Val Leu His Asp
                   165                 170                 175
   Ile Ser Ser Leu Leu Ser Arg His Lys His Leu Ala Lys Asp His Ser
               180                 185                 190
   Pro Asp Leu Tyr Ser Leu Glu Leu Ala Gly Leu Asp Glu Ile Gly Lys
           195                 200                 205
   Arg Tyr Gly Glu Asp Ser Glu Gln Phe Arg Asp Ala Ser Lys Ile Leu
       210                 215                 220
   Val Asp Ala Leu Gln Lys Phe Ala Asp Asp Met Tyr Ser Leu Tyr Gly
   225                 230                 235                 240
   Gly Asn Ala Val Val Glu Leu Val Thr Val Lys Ser Phe Asp Thr Ser
                   245                 250                 255
   Leu Ile Arg Lys Thr Arg Thr Ile Leu Glu Ala Lys Gln Ala Lys Asn
               260                 265                 270
   Pro Ala Ser Pro Tyr Asn Leu Ala Tyr Lys Tyr Asn Phe Glu Tyr Ser
           275                 280                 285
   Val Val Phe Asn Met
       290
<210> 3
   <211> 350
   <212> PRT
   <213> Homo sapiens
<220>
   <221> PEPTIDE
   <222> (1)..(350)
<400> 3
<210> 4
   <211> 16
   <212> PRT
   <213> Artificial Sequence
<220>
   <223> Epitope derived from SEQ ID NO:1 for monoclonal antobodies generation
<220>
   <221> PEPTIDE
   <222> (1)..(16)
<400> 4
   Val Phe Arg Asn Gly Asn Trp Pro Ile Pro Gly Glu Arg Ile Pro Asp
   1               5                   10                  15
<210> 5
   <211> 16
   <212> PRT
   <213> Artificial Sequence
<220>
   <223> Epitope derived from SEQ ID NO:1 for the generation of monoclonal antibodies
<220>
   <221> PEPTIDE
   <222> (1)..(16)
<400> 5
   Leu Arg Gln Leu Arg Asn Arg Leu Phe Gln Glu Asn Ser Val Leu Ser
   1 5 10 15
<210> 6
   <211> 15
   <212> PRT
   <213> Artificial Sequence
<220>
   <223> Epitope derived from SEQ ID NO:1 for the generation of monoclonal antibodies
<220>
   <221> PEPTIDE
   <222> (1)..(15)
<400> 6
   Arg His Lys His Leu Ala Lys Asp His Ser Pro Asp Leu Tyr Ser
   1 5 10 15
<210> 7
   <211> 15
   <212> PRT
   <213> Artificial Sequence
<220>
   <223> Epitope derived from SEQ ID NO:1 for the generation of monoclonal antibodies
<220>
   <221> PEPTIDE
   <222> (1)..(15)
<400> 7
   Glu Ile Gly Lys Arg Tyr Gly Glu Asp Ser Glu Gln Phe Arg Asp
   1 5 10 15
<210> 8
   <211> 13
   <212> PRT
   <213> Artificial Sequence
<220>
   <223> Epitope derived from SEQ ID NO:1 for the generation of monoclonal antibodies
<220>
   <221> PEPTIDE
   <222> (1)..(13)
<400> 8
   Val Lys Ser Phe Asp Thr Ser Leu Ile Arg Lys Thr Arg
   1               5                   10